# EUROPEAN PATENT APPLICATION

(11) **EP 4 835 062 A1**
(43) Date of publication of application: **04.11.2026**
(21) Application number: 24911151.9
(22) Date of filing: 25.12.2024
(51) Int. Cl.: C12N 9/00, C12N 1/15, C12N 1/21, C12P 21/00

(54) **MODIFIED AMINOACYL-TRNA SYNTHETASE, NUCLEIC ACID CONSTRUCT AND GENETICALLY ENGINEERED STRAIN**

(30) Priority: 25.12.2023 CN 202311806634
(71) Applicant: Kangma-Healthcode (Shanghai) Biotech Co., Ltd, Pudong New Area Shanghai 201321 (CN)
(72) Inventor: GUO, Min, Shanghai 201321 (CN); DING, Wenfei, Shanghai 201321 (CN); DAI, Tianchun, Shanghai 201321 (CN); FANG, Pengfei, Shanghai 201321 (CN); ZHENG, Li, Shanghai 201321 (CN); YU, Xue, Shanghai 201321 (CN)
(74) Representative: Lavoix
(86) International application number: PCT/CN2024/142051
(87) International publication number: WO 2025/140227

(57) **Abstract**

Provided are a modified aminoacyl-tRNA synthetase, a nucleic acid construct and a genetically engineered strain. The provided recombinant aminoacyl-tRNA synthetase can significantly improve the introduction efficiency of non-natural amino acids in IVTT regardless of exogenous addition or integration into cells.

## Description

### Technical Field

The present invention relates to the field of biotechnology, and preferably relates to an aminoacyl-tRNA synthetase, a nucleic acid construct thereof, a genetically engineered strain, and the application thereof in cell-free synthesis of proteins containing non-natural amino acids.

### Background Art

Whether for research on protein structure and function or the production of antibody-drug conjugates, there is a growing demand to introduce novel functional groups into polypeptide chains of proteins. By virtue of an orthogonal protein translation system, non-natural amino acids carrying specific chemical functional groups can be incorporated into polypeptide chains according to predefined genetic codons. Although the introduction of non-natural amino acids can be achieved intracellularly, cell-free *in vitro* protein translation systems possess irreplaceable advantages for large-scale production of proteins containing non-natural amino acids. This is due to competition with the canonical protein translation system, as well as the fact that the unique chemically reactive side-chain groups of non-natural amino acids often prevent them from freely crossing the cell membrane.

Since a cell-free *in vitro* protein translation system is not constrained by a cell membrane, the reaction can, in theory, be driven forward by increasing the concentration of the major components in the reaction system. An non-natural amino acid is first converted by a specific aminoacyl-tRNA synthetase into the corresponding aminoacyl-tRNA, which is then transported to the ribosome where, through codon pairing and under the catalysis of the ribosome, the non-natural amino acid is incorporated into the peptide chain. In this process, the binding affinity of the non-natural aminoacyl-tRNA for the transport protein and its compatibility with the ribosome are relatively weaker than those of the canonical aminoacyl-tRNA, which is unfavourable for the progression of the reaction. It is therefore necessary to promote the reaction by increasing the substrate concentration. The orthogonal aminoacyl-tRNA synthetase used for the incorporation of the non-natural amino acid is typically obtained by mutating the substrate-binding site of a natural aminoacyl-tRNA synthetase, and the resulting alteration in substrate specificity often comes at the cost of reduced catalytic activity. To compensate for this reduction in activity, it is necessary to use high concentrations of the enzyme, the tRNA, or the non-natural amino acid, so as to shift the reaction in the direction favorable for peptide chain synthesis.

The commonly used orthogonal translation system, PylRS-tRNA_{CUA}^{Py1}, are derived from the archaea *Methanosarcina mazei* and *Methanosarcina barkeri* PylRS (MmPylRS, MbPylRS). Due to its special structure, the recognition of tRNA by PylRS is independent of the anticodon loop, and it exhibits substrate recognition plasticity. Through directed evolution, the incorporation of more than 200 non-natural amino acids has been achieved using the PylRS-tRNA_{CUA}^{Py1} system.

Nevertheless, both MmPylRS and MbPylRS contain an N-terminal domain with low solubility, resulting in poor solubility thereof. Even after codon optimization, the expression level of MmPylRS and MbPylRS in *Escherichia coli* can hardly be improved, and the protein concentration cannot exceed 8 mg/ml after concentration. This fails to fully exploit the advantages of cell-free *in vitro* protein translation systems, and the incorporation efficiency of non-natural amino acids is very low, rendering them unsuitable for practical production applications.

*Methanomethylophilus alvus* PylRS (PylRS) is an aminoacyl-tRNA synthetase. This synthetase belongs to the nucleotide class, and its full name is pyrrolysyl-tRNA synthetase, commonly abbreviated as PylRS. The primary function of PylRS is to assist prokaryotes in synthesizing a special amino acid, pyrrolysine (Pyl), which is absent in all known eukaryotes ^{[1]}.

*Methanomethylophilus alvus* is a methanogenic archaeon natively isolated from the rumen of cattle. PylRS was discovered through sequence analysis of the *M. alvus* genome and can be used to engineer foreign proteins and enable them to exhibit new biological activities, thus possessing significant application value in the field of synthetic biology.

Recently, Chin et al. discovered a PylRS (MaPylRS) from *Methanomethylophilus* alvus. Compared with MmPylRS and MbPylRS, MaPylRS possesses a similar catalytic domain and tRNA binding domain, but lacks the N-terminal domain.

In current scientific research, MaPylRS is mainly applied to the engineering of foreign proteins in prokaryotes such as *Escherichia coli*^{[2]}, and there are also reports of successful application in eukaryotes ^{[3]}. According to the latest research results, when MaPylRS is expressed in *Saccharomyces cerevisiae* ^{[4]}, it can achieve efficient site-specific incorporation of target non-natural amino acids into a protein of interest. However, whether in *Escherichia coli* or *yeast,* MaPylRS exists in cells in the form of an exogenous plasmid, and there is no eukaryotic cell system in which MaPylRS is directly recombined into the genome thereof for stable and high-efficiency expression ^{[5]}.

Protein Expression Systems *in vitro* refers to a system for protein synthesis implemented under acellular conditions using cell lysates and the like. The synthesis of a target protein can be achieved merely by adding DNA or RNA templates, RNA polymerase, essential amino acids, ATP, cofactors and other components to the reaction system. Since the complex metabolic steps of an entire cell are not required, this system can achieve rapid and efficient large-scale protein production. Due to its high flexibility and simplified process, it has become one of the widely used tools in the biomedical field and scientific research ^{[6]}. Currently, the commercialized *in vitro* protein expression systems commonly used in experiments include the *Escherichia coli* extract system (ECE) ^{[7]}, rabbit reticulocyte lysate (RRL) ^{[8]}, wheat germ extract (WGE) ^{[9]}, insect cell extract (ICE) ^{[10]}, and human-derived systems ^{[11]}.

Owing to the absence of the poorly soluble N-terminal domain, MaPylRS can be expressed more readily in *Escherichia coli.* The final expression product can be concentrated to 40 mg/ml without precipitation, and thus is suitable for cell-free systems for non-natural amino acid introduction.

CRISPR/Cas (Clustered Regularly Interspaced Short Palindromic Repeats / CRISPR associated) is an immune system widely present in bacteria and archaea. As part of the defense mechanism of an organism, it can recognize and eliminate foreign DNA or RNA invading the host cell, and has been developed into an efficient and accurate gene editing tool ^{[12]}. This technology can precisely cut DNA, allowing researchers to add or delete specific parts of a gene to study their functions, and can also be used to treat certain genetic diseases ^{[13]}. When using the CRISPR/Cas9 system for gene editing, a specific gRNA sequence needs to be paired with the Cas9 nuclease protein and delivered into the target cells. After the gRNA binds to Cas9, it will be transported to the target genome. During the formation of the DNA double-strand break, the gRNA guides the Cas9 enzyme to find the PAM sequence (i.e., "protospacer adjacent motif") on the genome and recognize the 20 bp sequence upstream thereof. Then, the Cas9 enzyme creates a double-strand break at a position 3 bases upstream of the PAM. At the same time, if donor DNA is provided, the broken DNA strand can be ligated through the mechanism of homology-directed repair (HDR), thereby achieving the purpose of genetic modification ^{[14]}. There are many examples of genome engineering of *Saccharomyces cerevisiae (S. cerevisiae)* using the CRISPR/Cas9 system, including gene point mutations, gene knockouts, and gene insertions ^{[15, 16]}. For example, the pCAS plasmid widely used in the prior art carries both the Cas9 gene sequence and the gRNA element ^{[17]}, enabling genome engineering of *Saccharomyces cerevisiae* through a single transformation. *Kluyveromyces* is a genus of yeast fungi, belonging to a type of *Saccharomycetaceae* yeast. It has a wide range of industrial applications, especially in the food and beverage industry. *Kluyveromyces* is widely used in the fermentation process of food and beverages such as dairy products, wine, and beer. It can decompose sugars and produce various enzymes, promoting the fermentation process and imparting good taste and flavor to the food. Compared with other *Saccharomycetaceae* yeasts, *Kluyveromyces* possesses some unique characteristics, such as higher temperature adaptability and acid tolerance. This makes it more suitable for certain industrial applications under specific conditions, such as high-temperature fermentation and acidic environments. In addition, *Kluyveromyces* as a host system for expressing pharmaceutical proteins also has many advantages. First, through the design of a suitable gene expression vector and promoter sequence, *Kluyveromyces* can achieve high-level expression of exogenous genes. Second, *Kluyveromyces* possesses a complete set of protein folding and modification systems, capable of correctly folding complex pharmaceutical proteins and performing necessary glycosylation modifications. Third, *Kluyveromyces* is easy to culture and can perform intracellular soluble expression, thereby facilitating the extraction and purification of the target protein, with lower engineering production costs. Overall, *Kluyveromyces* is a host system with great potential that can be used for the efficient expression of pharmaceutical proteins.

### References

1. Yanagisawa T, Seki E, Tanabe H, Fujii Y, Sakamoto K, Yokoyama S. Crystal Structure of Pyrrolysyl-tRNA Synthetase from a Methanogenic Archaeon ISO4-G1 and Its Structure-Based Engineering for Highly-Productive Cell-Free Genetic Code Expansion with Non-Canonical Amino Acids. Int J Mol Sci. 2023 Mar 26;24(7):6256. doi: 10.3390/ijms24076256. PMID: 37047230; PMCID: PMC10094482.
2. Kobayashi T, Yanagisawa T, Sakamoto K, Yokoyama S. Recognition of non-alpha-amino substrates by pyrrolysyl-tRNA synthetase. J Mol Biol. 2009 Feb 6;385(5):1352-60. doi: 10.1016/j.jmb.2008.11.059. Epub 2008 Dec 11. PMID: 19100747.
3. Beránek V, Willis JCW, Chin JW. An Evolved Methanomethylophilus alvus Pyrrolysyl-tRNA Synthetase/tRNA Pair Is Highly Active and Orthogonal in Mammalian Cells. Biochemistry. 2019 Feb 5;58(5):387-390. doi: 10.1021/acs.biochem.8b00808. Epub 2018 Sep 27. PMID: 30260626; PMCID: PMC6365905.
4. Stieglitz JT, Lahiri P, Stout MI, Van Deventer JA. Exploration of Methanomethylophilus alvus Pyrrolysyl-tRNA Synthetase Activity in Yeast. ACS Synth Biol. 2022 May 20;11(5):1824-1834. doi: 10.1021/acssynbio.2c00001. Epub 2022 Apr 13. PMID: 35417129; PMCID: PMC10112046.
5. Avila-Crump S, Hemshorn ML, Jones CM, Mbengi L, Meyer K, Griffis JA, Jana S, Petrina GE, Pagar VV, Karplus PA, Petersson EJ, Perona JJ, Mehl RA, Cooley RB. Generating Efficient Methanomethylophilus alvus Pyrrolysyl-tRNA Synthetases for Structurally Diverse Non-Canonical Amino Acids. ACS Chem Biol. 2022 Dec 16;17(12):3458-3469. doi: 10.1021/acschembio.2c00639. Epub 2022 Nov 16. PMID: 36383641; PMCID: PMC9833845.
6. Craig D, Howell MT, Gibbs CL, Hunt T, Jackson RJ. Plasmid cDNA-directed protein synthesis in a coupled eukaryotic in vitro transcription-translation system. Nucleic Acids Res. 1992 Oct 11;20(19):4987-95. doi: 10.1093/nar/20.19.4987. PMID: 1383935; PMCID: PMC334274.
7. Kim DM, Kigawa T, Choi CY, Yokoyama S. A highly efficient cell-free protein synthesis system from Escherichia coli. Eur J Biochem. 1996 Aug 1;239(3):881-6. doi: 10.1111/j.1432-1033.1996.0881u.x. PMID: 8774739.
8. Jackson RJ, Hunt T. Preparation and use of nuclease-treated rabbit reticulocyte lysates for the translation of eukaryotic messenger RNA. Methods Enzymol. 1983;96:50-74. doi: 10.1016/s0076-6879(83)96008-1. PMID: 6656641.
9. Erickson AH, Blobel G. Cell-free translation of messenger RNA in a wheat germ system. Methods Enzymol. 1983;96:38-50. doi: 10.1016/s0076-6879(83)96007-x. PMID: 6656637.
10. Ezure T, Suzuki T, Higashide S, Shintani E, Endo K, Kobayashi S, Shikata M, Ito M, Tanimizu K, Nishimura O. Cell-free protein synthesis system prepared from insect cells by freeze-thawing. Biotechnol Prog. 2006 Nov-Dec;22(6):1570-7. doi: 10.1021/bp060110v. PMID: 17137303.
11. Mikami S, Kobayashi T, Masutani M, Yokoyama S, Imataka H. A human cell-derived in vitro coupled transcription/translation system optimized for production of recombinant proteins. Protein Expr Purif. 2008 Dec;62(2):190-8. doi: 10.1016/j.pep.2008.09.002. Epub 2008 Sep 11. PMID: 18814849.
12. Sander JD, Joung JK. CRISPR-Cas systems for editing, regulating and targeting genomes. Nat Biotechnol. 2014 Apr;32(4):347-55. doi: 10.1038/nbt.2842. Epub 2014 Mar 2. PMID: 24584096; PMCID: PMC4022601.
13. Jinek M, Chylinski K, Fonfara I, Hauer M, Doudna JA, Charpentier E. A programmable dual-RNA-guided DNA endonuclease in adaptive bacterial immunity. Science. 2012 Aug 17;337(6096):816-21. doi: 10.1126/science.1225829. Epub 2012 Jun 28. PMID: 22745249; PMCID: PMC6286148.
14. Bibikova M, Carroll D, Segal DJ, Trautman JK, Smith J, Kim YG, Chandrasegaran S. Stimulation of homologous recombination through targeted cleavage by chimeric nucleases. Mol Cell Biol. 2001 Jan;21(1):289-97. doi: 10.1128/MCB.21.1.289-297.2001. PMID: 11113203; PMCID: PMC88802.
15. DiCarlo JE, Norville JE, Mali P, Rios X, Aach J, Church GM. Genome engineering in Saccharomyces cerevisiae using CRISPR-Cas systems. Nucleic Acids Res. 2013 Apr;41(7):4336-43. doi: 10.1093/nar/gkt135. Epub 2013 Mar 4. PMID: 23460208; PMCID: PMC3627607.
16. Bao Z, Xiao H, Liang J, Zhang L, Xiong X, Sun N, Si T, Zhao H. Homology-integrated CRISPR-Cas (HI-CRISPR) system for one-step multigene disruption in Saccharomyces cerevisiae. ACS Synth Biol. 2015 May 15;4(5):585-94. doi: 10.1021/sb500255k. Epub 2014 Sep 19. PMID: 25207793.
17. Ryan OW, Skerker JM, Maurer MJ, Li X, Tsai JC, Poddar S, Lee ME, DeLoache W, Dueber JE, Arkin AP, Cate JH. Selection of chromosomal DNA libraries using a multiplex CRISPR system. Elife. 2014 Aug 19;3:e03703. doi: 10.7554/eLife.03703. PMID: 25139909; PMCID: PMC4161972.

### Summary of Invention

In order to overcome the deficiencies of the prior art, the present invention designs a histidine tag at the N-terminus and the C-terminus of the MaPylRS gene, respectively, as well as a thrombin cleavage site for convenient tag removal. The results show that adding sequences at the N-terminus or C-terminus not only promotes the stability of the enzyme and facilitates purification, but also further improves the catalytic activity of the enzyme.

In addition, in existing literature reports and commercial kits, the MaPylRS protein is manually added exogenously, or a plasmid containing the expression construct thereof is introduced by transformation/transfection or the like. This has adverse effects on the interpretation of experimental results, the complexity and stability of experimental results, and the production cost of the target protein. By adopting the efficient CRISPR/Cas9 gene editing system, the present invention integrates the MaPylRS gene into the genome of *Kluyveromyces,* achieving stable and efficient expression of the MaPylRS gene in yeast without the need for antibiotic screening and maintenance. On this basis, the insertion of non-natural amino acids into target proteins is realized.

The first aspect of the present invention provides a recombinant aminoacyl-tRNA synthetase having the structure represented by Formula I:

A1-A2-A3-A4 (I);

in Formula I,
A1 is absent or a histidine tag;
A2 is absent or a thrombin cleavage site;
A3 is absent or a tag protein;
A4 is an aminoacyl-tRNA synthetase;
"-" is independently a bond or an amino acid linker sequence,
and at least one of A1 ~ A3 is present,
and the connections of A1 ~ A4 can be either from N-terminus to C-terminus or from C-terminus to N-terminus.

Further preferably, the aminoacyl-tRNA synthetase is selected from a natural or mutated Pyl-tRNA synthetase (PylRS), Leu-tRNA synthetase (LeuRS), Tyr-tRNA synthetase (TyrRS), Phe-tRNA synthetase (PheRS), or Trp-tRNA synthetase (TrpRS).

Further preferably, the aminoacyl-tRNA synthetase is selected from a natural or mutated MaPylRS, MmPylRS, MbPylRS, EcTyrRS, MjTyrRS, EcLeuRS, ScPheRS, ScTrpRS, or BsTrpRS.

Further preferably, the aminoacyl-tRNA synthetase is natural or mutated MaPylRS.

Further preferably, the sequence of the aminoacyl-tRNA synthetase is set forth in SEQ ID NO:60.

Further preferably, the aminoacyl-tRNA synthetase comprises the sequence set forth in SEQ ID NO:60 or an active fragment thereof, or is a polypeptide having ≥85% homology (preferably, ≥90% homology; more preferably, ≥95% homology; most preferably, ≥97% homology, such as 98% or more, 99% or more) to the amino acid sequence set forth in SEQ ID NO:60 and having the same activity as the sequence of SEQ ID NO:60.

Further preferably, the structure of the histidine tag is n×His, wherein 1≤n≤50; preferably 2≤n≤30; further preferably, 5≤n≤20; more preferably 6≤n≤10.

Further preferably, the tag protein is selected from a T7 tag, a CBP tag, a c-Myc tag, a FLAG tag, a Spot tag, a C tag, an Avi tag, a Strep tag, a SUMO tag, a GST tag, an MBP tag, or a combination thereof; preferably a T7 tag.

Further preferably, the A1-A2-A3 is linked to the N-terminus or C-terminus of A4.

Further preferably, the His tag is linked to the N-terminus or C-terminus of the aminoacyl-tRNA synthetase.

Further preferably, at the N-terminus of the aminoacyl-tRNA synthetase, the thrombin cleavage site and the tag protein are linked in the order from N-terminus to C-terminus.

Further preferably, at the N-terminus of the aminoacyl-tRNA synthetase, the His tag, the thrombin cleavage site, and the tag protein are linked in the order from N-terminus to C-terminus.

Further preferably, the amino acid sequence of the recombinant aminoacyl-tRNA synthetase is selected from any one or more of the following: SEQ ID NO:1 ~ 3 or SEQ ID NO:64.

Further preferably, the recombinant aminoacyl-tRNA synthetase comprises the sequence set forth in any one of SEQ ID NOs: 1 ~ 3, and SEQ ID NO:64, or an active fragment thereof, or is a polypeptide respectively having ≥85% homology (preferably, ≥90% homology; more preferably, ≥95% homology; most preferably, ≥97% homology, such as 98% or more, 99% or more) to the amino acid sequence set forth in any one of SEQ ID NOs: 1 ~ 3, and SEQ ID NO:64 and having the same activity as the sequence of any one of SEQ ID NOs: 1 ~ 3, and SEQ ID NO:64.

Further preferably, the coding sequence of the recombinant aminoacyl-tRNA synthetase is selected from any one or more of the following: SEQ ID NO:4, SEQ ID NO:61, SEQ ID NO:62, or SEQ ID NO:63.

Further preferably, the coding sequence of the recombinant aminoacyl-tRNA synthetase comprises the sequence set forth in any one of SEQ ID NO:4, SEQ ID NO:61, SEQ ID NO:62, and SEQ ID NO:63, or an active fragment thereof, or is a nucleotide sequence respectively having ≥85% homology (preferably, ≥90% homology; more preferably, ≥95% homology; most preferably, ≥97% homology, such as 98% or more, 99% or more) to the nucleotide sequence set forth above and respectively having the same activity as the sequence of any one of SEQ ID NO:4, SEQ ID NO:61, SEQ ID NO:62, or SEQ ID NO:63.

The second aspect of the present invention provides a nucleic acid construct encoding the recombinant aminoacyl-tRNA synthetase according to the first aspect of the present invention.

Further preferably, the nucleic acid construct at least comprises a nucleic acid sequence having the structure represented by Formula II: Z1-Z2-Z3-Z4, wherein Z1 ~ Z4 are respectively elements for constituting the construct; "-" is independently a bond or a nucleotide linker sequence; Z1 is absent or a coding sequence for the histidine tag, Z2 is absent or a coding sequence for the thrombin cleavage site, Z3 is absent or the coding sequence for the tag protein, and Z4 is a coding sequence for the aminoacyl-tRNA synthetase; wherein at least one of Z1 ~ Z3 is present.

The structure of Formula II may be either from 5' to 3' or from 3' to 5';
that is, the encoded product of Z1-Z2-Z3 is linked to the N-terminus or C-terminus of the encoded product of Z4.

More preferably, the encoded product of Z1-Z2-Z3 is linked to the N-terminus of the encoded product of Z4.

Further preferably, the amino acid sequence encoded by Z1 is HHHHHH; more preferably, Z1 encodes a sequence comprising HHHHHH or an active fragment thereof, or is a nucleotide sequence having ≥85% homology (preferably, ≥90% homology; more preferably, ≥95% homology; most preferably, ≥97% homology, such as 98% or more, 99% or more) to the nucleotide sequence set forth above and having the same activity as the above sequence.

Further preferably, the amino acid sequence encoded by Z2 is LVPRGS; more preferably, Z2 encodes a sequence comprising LVPRGS or an active fragment thereof, or is a nucleotide sequence having ≥85% homology (preferably, ≥90% homology; more preferably, ≥95% homology; most preferably, ≥97% homology, such as 98% or more, 99% or more) to the nucleotide sequence set forth above and having the same activity as the above sequence.

Further preferably, the amino acid sequence encoded by Z3 is SEQ ID NO:59; more preferably, Z3 encodes a sequence comprising the sequence set forth in SEQ ID NO:59 or an active fragment thereof, or is a nucleotide sequence having ≥85% homology (preferably, ≥90% homology; more preferably, ≥95% homology; most preferably, ≥97% homology, such as 98% or more, 99% or more) to the nucleotide sequence set forth above and having the same activity as the above sequence.

Further preferably, the nucleic acid construct further comprises a promoter.

Further preferably, the nucleic acid construct has the structure represented by Formula III:

Z5-Z1-Z2-Z3-Z4.

Further preferably, the promoter is selected from PGK1, GAP1, ADH1, HXK1, GAPDH1, TEF1, or TIF11.

The third aspect of the present invention provides a vector, wherein the vector comprises the nucleic acid construct according to the second aspect of the present invention.

The fourth aspect of the present invention provides a genetically engineered strain, wherein one or more sites of the genome of the genetically engineered strain are integrated with the nucleic acid construct according to the second aspect of the present invention.

Further preferably, the site is selected from Lys1-5, glpA, or UPF1.

Further preferably, the nucleic acid construct further comprises a promoter and a terminator.

Further preferably, the promoter is selected from PGK1, GAP1, ADH1, HXK1, GAPDH1, TEF1, TIF11, GAL1, GAL7, or GAL10.

Further preferably, the terminator is selected from CYC1, GPM1, TDH2, or ACT1.

Further preferably, the genetically engineered strain comprises the recombinant aminoacyl-tRNA synthetase according to the first aspect of the present invention.

Further preferably, the genetically engineered strain comprises the vector according to the third aspect of the present invention.

Further preferably, the strain is derived from mammalian cells, plant cells, yeast cells, insect cells, prokaryotic cells, or any combination thereof.

The fifth aspect of the present invention provides a method for synthesizing a protein incorporated with a non-natural amino acid, wherein the recombinant aminoacyl-tRNA synthetase according to the first aspect of the present invention is used, or the genetically engineered strain according to the fourth aspect of the present invention is used to provide the recombinant aminoacyl-tRNA synthetase.

The sixth aspect of the present invention provides a cell-free system for synthesizing a protein containing a non-natural amino acid, characterized in that the cell-free system at least comprises: (a) a cell extract, and (b) one or more of the recombinant aminoacyl-tRNA synthetase according to the first aspect of the present invention, the nucleic acid construct according to the second aspect of the present invention, or the vector according to the third aspect of the present invention; wherein the cell extract is derived from mammalian cells, plant cells, yeast cells, insect cells, prokaryotic cells, or any combination thereof.

The seventh aspect of the present invention provides a cell-free system for synthesizing a protein containing a non-natural amino acid, characterized in that the cell-free system at least comprises a cell extract, and the cell extract is derived from the genetically engineered strain according to the fourth aspect of the present invention.

Further preferably, the cell extract is selected from any one source or combination of the following: *Escherichia coli, Kluyveromyces lactis,* wheat germ cells, insect cells, rabbit reticulocytes, CHO cells, COS cells, VERO cells, BHK cells, human fibrosarcoma HT1080 cells, or a combination thereof.

Further preferably, the cell extract is derived from a yeast cell.

Further, the yeast cell is selected from *Pichia pastoris, Pichia finlandica, Pichia trehalophila, Pichia koclamae, Pichia membranaefaciens, Pichia minuta, Ogataea minuta, Pichia lindneri, Pichia opuntiae, Pichia thermotolerans, Pichia salictaria, Pichia guercuum, Pichia pijperi, Pichia stiptis, Pichia methanolica, Pichia* sp., *Saccharomyces cerevisiae,* beer yeast, sugarcane molasses yeast, *Saccharomyces* sp., *Hansenula polymorpha, Candida utilis, Kluyveromyces,* or a combination thereof.

Further, more preferably, the *Kluyveromyces* further comprises: *Kluyveromyces lactis (K. lactis), Kluyveromyces marxianus, Kluyveromyces dobzhanskii, Kluyveromyces aestuarii, Kluyveromyces nonfermentans, Kluyveromyces wickerhamii, Kluyveromyces thermotolerans, Kluyveromyces fragilis, Kluyveromyces hubeiensis, Kluyveromyces polysporus, Kluyveromyces siamensis, Kluyveromyces yarrowii,* or a combination thereof; preferably, the yeast cell is a *Kluyveromyces* cell, more preferably a *Kluyveromyces lactis* cell.

Further preferably, the cell-free system further comprises: a non-natural amino acid, an orthogonal tRNA, and a template comprising a gene sequence of a protein of interest, wherein a codon encoding an amino acid in the gene sequence of the protein of interest is mutated.

Further preferably, the non-natural amino acid has the structural formula of compound (2) or a salt form thereof:

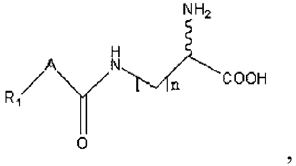

Wherein n is a natural number ranging from 1 to 20; R₁ is selected from substituted or unsubstituted C5-C60 aryl or heteroaryl , substituted or unsubstituted C1-C20 alkyl , substituted or unsubstituted C2-C20 alkenyl , or substituted or unsubstituted C2-C20 alkynyl ; A is selected from O or -CH₂-.

In another preferred embodiment, n is selected from 1, 2, 3, 4, 5, 6, 7, 8, 9, 10, 11, 12, 13, 14, 15, 16, 17, 18, 19, or 20.

In another preferred embodiment, n is a natural number ranging from 1 to 10.

In another preferred embodiment, n is selected from 1, 2, 3, 4, 5, 6, 7, 8, 9, or 10.

In another preferred embodiment, n is a natural number ranging from 1 to 6.

In another preferred embodiment, n is selected from 1, 2, 3, 4, 5, or 6.

In another preferred embodiment, R₁ is selected from substituted or unsubstituted C5-C30 aryl or heteroaryl .

In another preferred embodiment, R₁ is a substituted or unsubstituted phenyl group.

In another preferred embodiment, R₁ is selected from substituted or unsubstituted C2-C20 alkenyl .

In another preferred embodiment, R₁ is selected from substituted or unsubstituted C2-C10 alkenyl .

In another preferred embodiment, R₁ is selected from substituted or unsubstituted C2-C6 alkenyl .

In another preferred embodiment, R₁ is selected from substituted or unsubstituted C2-C20 alkynyl .

In another preferred embodiment, R₁ is selected from substituted or unsubstituted C2-C10 alkynyl .

In another preferred embodiment, R₁ is selected from substituted or unsubstituted C2-C6 alkynyl .

In another preferred embodiment, A is O.

In another preferred embodiment, A is -CH₂-.

In another preferred embodiment, the substituent is a common substituent group in the art, such as aryl, heteroaryl, alkyl, cycloalkyl, aryloxy, heteroaryloxy, alkyloxy, cycloalkyloxy, hydroxyl, mercapto, ester, carboxyl, cyano, halogen, nitro, sulfonic acid, azido, alkenyl, alkynyl, phosphate, and the like.

In another preferred embodiment, the non-natural amino acid has a structural formula selected from one or a combination of the following, or a salt form thereof:

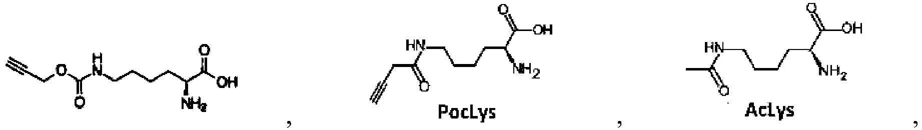

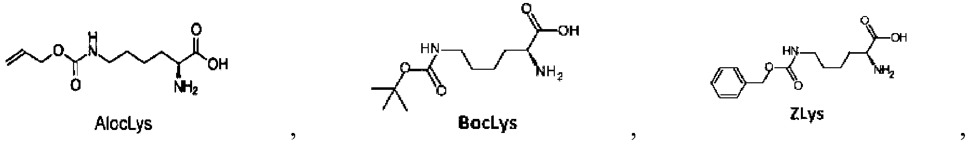

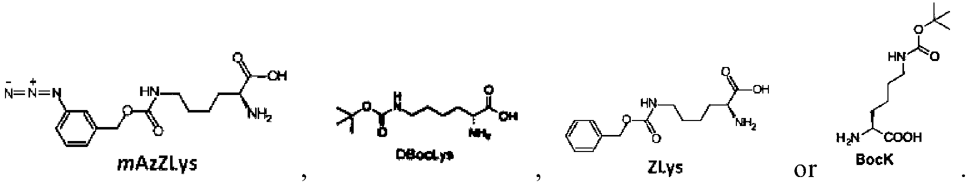

Further preferably, the protein of interest is selected from: a fluorescein protein, a luciferase (such as firefly luciferase), a fluorescent protein (such as green fluorescent protein, yellow fluorescent protein), an aminoacyl-tRNA synthetase, glyceraldehyde-3-phosphate dehydrogenase, catalase, actin, a variable region of an antibody, a luciferase mutant, α-amylase, enterocin A, hepatitis C virus E2 glycoprotein, an insulin precursor, interferon αA, a cytokine, interferon α2b, interleukin-1β, lysozyme, serum albumin, a single-chain antibody fragment (scFv), transthyretin, tyrosinase, xylanase, or a combination thereof.

Further preferably, the protein of interest comprises a wild-type protein, a mutant protein, or a recombinant protein.

The eighth aspect of the present invention provides a method for preparing the genetically engineered strain according to the fourth aspect of the present invention, characterized by introducing or integrating the nucleic acid construct according to the second aspect of the present invention into a cell by transformation, transfection, or gene editing technology.

Further preferably, the nucleic acid construct according to the second aspect of the present invention is integrated into the genome of the cell by gene editing technology.

Further preferably, the nucleic acid construct is integrated into the genome of cells via active site integration.

Further preferably, the nucleic acid construct further comprises a promoter and a terminator.

Further preferably, the promoter is selected from PGK1, GAP1, ADH1, HXK1, GAPDH1, TEF1, or TIF11.

Further preferably, the terminator is selected from CYC1, GPM1, TDH2, or ACT1.

Further preferably, the site is selected from Lys1-5, glpA, or UPF1.

The ninth aspect of the present invention provides a kit, characterized in that the kit comprises the system according to the sixth aspect or the seventh aspect of the present invention.

The tenth aspect of the present invention provides a method for *in vitro* synthesis of a protein containing a non-natural amino acid, characterized by using the cell-free system according to the sixth aspect or the seventh aspect of the present invention or the kit according to the ninth aspect of the present invention for preparation.

The advantages of the present invention are as follows:
(1) The design of a structure comprising the His tag at the N-terminus and C-terminus of a natural or modified MaPylRS synthetase, respectively, further promotes the stability of the enzyme, facilitates purification, improves the catalytic activity of the enzyme, and is beneficial for improving the incorporation efficiency of non-natural amino acids.
(2) The linking of the His tag, the T7 tag, and the thrombin cleavage site at the N-terminus of a natural or modified MaPylRS synthetase further promotes the stability of the enzyme, facilitates purification, improves the catalytic activity of the enzyme, and is beneficial for improving the incorporation efficiency of non-canonical amino acids.
(3) The linking of the T7 tag and the thrombin cleavage site at the N-terminus of a natural or modified MaPylRS synthetase further promotes the stability of the enzyme, facilitates purification, improves the catalytic activity of the enzyme, and is beneficial for improving the incorporation efficiency of non-natural amino acids.
(4) In the present invention, the designed recombinant expression construct of MaPylRS is integrated into the cell genome through CRISPR/Cas9, combined with efficient transformation technology, thereby achieving stable presence of MaPylRS in the cell genome and continuous expression of the MaPylRS protein.
(5) The preparation of a *Kluyveromyces* strain with the inserted recombinant MaPylRS into an in vitro expression system achieves site-specific non-natural amino acid incorporation into an exogenous target protein, greatly simplifies the preparation steps, saves costs, and increases the stability of the protein synthesized and incorporated with non-natural amino acid.

### Brief Description of Drawings

Figure 1 shows a construction diagram of long N-terminal MaPylRS.
Figure 2 shows a construction diagram of N-His MaPylRS.
Figure 3 shows a construction diagram of C-His MaPylRS.
Figure 4 shows an electrophoresis image of the lysed supernatant of the three proteins after induction, wherein 1 represents long N-terminal MaPylRS, 2 represents N-His MaPylRS, and 3 represents C-His MaPylRS. The MaPylRS protein with a longer N-terminus exhibits a higher expression level and higher solubility of the protein. The protein concentration can reach up to 120 mg/mL (3.4 mM).
Figure 5 shows a comparison of the activity of the proteins expressed by the three forms of constructs for incorporating a non-naturall amino acid at the same concentration. All three forms of MaPylRS can catalyze the expression of a protein containing a non-natural amino acid. In the figure, inverted triangles represent groups without addition of non-natural amino acids, and dots represent groups with addition of non-natural amino acids. nhis represents N-His MaPylRS; chis represents C-His MaPylRS; N-terminal represents long N-terminal MaPylRS.
Figure 6 shows a comparison of the activity of the native MaPylRS (referring to the non-recombinant MaPylRS) and the recombinant MaPylRS (long N-terminal MaPylRS and N-His MaPylRS) for incorporating a non-natural amino acid. In the figure, nhis represents N-His MaPylRS, long terminal represents long N-terminal MaPylRS. In each comparative bar, the left part represents groups with the addition of a non-natural amino acid, and the right part represents groups with the addition of a non-natural amino acid.
Figure 7 shows a comparison of the RFP/GFP ratio of the native MaPylRS and the recombinant MaPylRS (long N-terminal MaPylRS and N-His MaPylRS) for incorporating a non-natural amino acid. In the figure, nhis represents N-His MaPylRS, long terminal represents long N-terminal MaPylRS, and no tag represents the native MaPylRS.
Figure 8 shows a schematic diagram of the structure of plasmid pKM-CAS1.0-KlLys1-5.
Figure 9 shows a schematic diagram of the structure of plasmid pKM-MaPylRS.
Figure 10 shows a schematic diagram of the structure of plasmid pKM-CAS1.0-KlglpA.
Figure 11 shows a schematic diagram of the structure of plasmid pKM-CAS1.0-KlUPF1.
Figure 12 shows the RFP activity measured in Example 9.
Figure 13 shows the GFP activity measured in Example 9.
Figure 14 shows the RFP/GFP ratio measured in Example 9.
Figure 15 shows the GFP activity measured in Example 10.
Figure 16 shows the RFP activity measured in Example 10.
Figure 17 shows the RFP/GFP ratio measured in Example 10.

In the drawings of the present application, the labels "non" or "control" indicate groups where no non-natural amino acid is added to the reaction system.

### Detailed Description

It should be noted that the embodiments and the features disclosed therein of the present application may be combined with each other without conflict.

The present invention will be further described in detail below in conjunction with the specific embodiments and examples. It shall be understood that these examples are merely intended to illustrate the present invention, rather than to limit the scope thereof. For experimental methods without specified conditions in the following examples, the conditions specified in the aforementioned detailed description shall be adopted preferentially; otherwise, conventional conditions or conditions recommended by the manufacturer shall be applied.

Unless otherwise specified, all percentages and parts referred to in the present invention are weight percentages and weight parts.

Unless otherwise specified, the materials and reagents used in the embodiments of the present invention are all commercially available products.

Unless otherwise specified, the unit of temperature in the present application is degrees Celsius (°C).

### Nouns and Terms

The following is an explanation or illustration of the meanings of some relevant "nouns" and "terms" used in the present invention, in order to facilitate a better understanding of the present invention. The corresponding explanations or illustrations apply to the entirety of the present invention, both the text that follows and the text that precedes. When the present invention involves cited documents, the definitions of related terms, nouns, and phrases in the cited documents are also incorporated by reference; however, where they conflict with the definitions in the present invention, the definitions in the present invention shall prevail. Such conflict shall not affect that the components, substances, compositions, materials, systems, formulations, species, methods, apparatuses and the like determined in the cited literatures shall be implemented in accordance with the contents of the cited literatures.

In the present invention, preferred implementations such as "preferably", "better", "more preferably", "preferred", "further preferably", and the like shall not impose any limitation on the scope of the invention and the scope of protection in any sense, are not intended to limit the scope and implementations of the present invention, and are provided merely as some implementations by way of example.

In the description of the present invention, the following expressions shall not impose any limitation on the scope of the invention and the scope of protection in any sense: preferred forms such as "one of the preferred", "one of the preferred options", "one of the preferred embodiments", "one of the preferred examples", "preferred example", "in a preferred embodiment", "in some preferred examples", "in some preferred embodiments", "preferred for", "preferably", "preferred", "more preferably", "better", "further preferably", "most preferably", and the like; and illustrative enumerations such as "one of the embodiments", "one of the examples", "for example", "specific example", "as an example", "by way of example", "such as", "for instance", "like" and the like. Moreover, the specific features described in each form are included in at least one specific implementation of the present invention. In the present invention, the specific features described in each form may be combined in any suitable method in any one or more specific implementations. In the present invention, the technical features or technical solutions corresponding to each preferred form may also be combined in any suitable method.

In the present invention, "any combination thereof" denotes "greater than 1" in quantity and, in terms of the scope of coverage, denotes a group consisting of the following situations: "any one selected therefrom, or any at least two selected therefrom."

In the present invention, descriptions of "one or more," such as "one or more," "one or more kinds of," and the like, have the same meaning as "at least one," "at least one kind of," "a combination thereof," "or a combination thereof," "and a combination thereof," "or any combination thereof," "and any combination thereof," and the like, and may be used interchangeably, denoting a quantity equal to "1" or "greater than 1."

In the present invention, "or/and" and "and/or" are used to denote "any one selected therefrom or any combination thereof," and also denote at least one thereof.

The term "about" refers to a value or composition falling within an acceptable error range of a specific value or composition as determined by a person skilled in the art, which depends partially on the method for measuring or determining the value or composition. For example, the expression "about 100" as used herein encompasses all values between 99 and 101 (e.g., 99.1, 99.2, 99.3, 99.4 and so forth).

Sequence identity (or homology) is determined by aligning two sequences over a predetermined comparison window (which may be 50%, 60%, 70%, 80%, 90%, 95%, or 100% of the length of the reference nucleotide sequence or protein) and determining the number of positions at which identical residues occur. Typically, this is expressed as a percentage. Methods for measuring the sequence identity of nucleotide sequences are well known to those skilled in the art.

The prior art means described in the present invention by terms such as "conventional," "routine," "general," "common," "frequent," and the like are also incorporated by reference as part of the content of the present invention. Unless otherwise specified, such prior art means may be regarded as one of the preferred implementations of part of the technical features of the present invention, and shall not limit the scope and protection scope of the present invention in any way.

All documents mentioned in the present invention and the documents directly or indirectly cited by these documents are incorporated by reference into the present application as if each individual document were individually incorporated by reference.

It should be understood that within the scope of the present invention, the various technical features of the present invention described above and those specifically described hereinafter (including but not limited to the embodiments) may be combined with each other to form new or preferred technical solutions, provided that they can be used to implement the present invention. Due to space limitations, they are not exhaustively described one by one.

*In vitro* protein synthesis reaction refers to a reaction for synthesizing a protein in an *in vitro* cell-free synthesis system, which at least comprises a translation process. *In vitro* protein synthesis reaction includes, but is not limited to, an IVT reaction *(in vitro* translation reaction), an IVTT reaction *(in vitro* transcription-translation reaction), and an IVDTT reaction (*in vitro* replication-transcription-translation reaction). In the present invention, an IVTT reaction is preferred. The IVTT reaction, corresponding to the IVTT system, is a process of transcribing and translating DNA into a protein *in vitro.* Therefore, we also refer to this type of *in vitro* protein synthesis system as a D2P system, a D-to-P system, a D_to_P system, or a DNA-to-Protein system. The corresponding *in vitro* protein synthesis method is also referred to as a D2P method, a D-to-P method, a D_to_P method, or a DNA-to-Protein method.

"Cell-free system" means that during *in vitro* protein synthesis, it is not carried out by means of secretion and expression by intact cells. It should be noted that in the *in vitro* cell-free protein synthesis system of the present invention, the addition of cellular components to promote the reaction is also permitted, but the added cells do not have the secretion and expression of the exogenous target protein as their primary purpose. In addition, a small number of intact cells may be intentionally added to the intact-cell-free D2P system constructed in accordance with the present invention (for example, the protein content provided by the cells does not exceed 30 wt% compared to the protein content provided by the cell extract). Such an "evasive" approach is also encompassed within the scope of protection of the present invention.

Protein of interest is the target expression product of the *in vitro* protein synthesis system of the present invention, which is not synthesized and secreted by a host cell, but is synthesized *in vitro* based on an exogenous nucleic acid template, and may also be referred to as an exogenous protein. The exogenous protein may be a protein, a fusion protein, a protein-containing molecule, or a mixture of fusion protein molecules; it also broadly includes polypeptides. The product obtained after performing the *in vitro* protein synthesis reaction based on the nucleic acid template encoding the target protein may be a single substance, or may be a combination of two or more substances. "Exogenous protein," "protein of interest," "target protein," and "target translation product" have the same meaning and may be translated as "objective protein," "interested protein," "objective translated product," "interested protein product," and the like, and are used interchangeably in the present invention.

D2P means DNA-to-Protein, referring to the process from a DNA template to a protein product. For example, D2P technology, D2P system, D2P method, D2P kit, and the like.

"Expression system of the present invention", *"in vitro* expression system of the present invention", *"in vitro* cell-free expression system" and *"in vitro* cell-free expression system" are used interchangeably and all refer to the *in vitro* protein expression system of the present invention. Other descriptive terms may also be used, such as: protein *in vitro* synthesis system, *in vitro* protein synthesis system, cell-free system, cell free system, cell-free protein synthesis system, cell-free *in vitro* protein synthesis system, *in vitro* cell-free protein synthesis system, *in vitro* cell-free synthesis system, CFS system (cell-free system), CFPS system (cell-free protein synthesis system), and the like. According to the reaction mechanism, it may include an *in vitro* translation system (which may be abbreviated as an IVT system, an mR2P system), an *in vitro* transcription-translation system (which may be abbreviated as an IVTT system, a D2P system), an *in vitro* replication-transcription-translation system (which may be abbreviated as an IVDTT system, a D2P system), and the like. In the present invention, the IVTT system is preferred. We also refer to the *in vitro* protein synthesis system as a "Protein Factory" (or "proteinfactory" or "Proteinfactory"). The *in vitro* protein synthesis system provided by the present invention is described in an open-ended method with respect to its components. The cell-free protein synthesis system of the present invention achieves the *in vitro* synthesis of the target protein by using exogenous DNA, mRNA, or a combination thereof as the nucleic acid template for protein synthesis, and by artificially controlling and supplementing substrates required for protein synthesis and substances such as transcription and translation-related protein factors.

In the present invention, the two expressions of protein have the same meaning, both are translated as protein and may be used interchangeably.

In the present invention, the two expressions of system are both translated as system and may be used interchangeably.

In the present invention, "amount of protein synthesized", "amount of protein expressed", and "yield of protein expression" have the same meaning and may be used interchangeably.

In the present invention, cell extract, cell extract solution, cell lysate, cell disruption product, and cell lysis product have the same meaning and may be used interchangeably. English descriptive terms such as cell extract, cell lysate, and the like may be used.

In the present invention, energy system, the energy system, and energy supply system have the same meaning and may be used interchangeably. Energy regeneration system and the energy regeneration system have the same meaning and may be used interchangeably. The energy regeneration system is a preferred implementation or a component of the energy system.

Further, the present invention provides a cell-free protein synthesis system, at least comprising a cell extract or a cell lysate.

Further preferably, the cell-free protein synthesis system further comprises one or more components selected from the group consisting of: a substrate for synthesizing RNA, a substrate for synthesizing a protein, polyethylene glycol or an analog thereof, magnesium ions, potassium ions, a buffer, an RNA polymerase, an energy regeneration system, dithiothreitol, and optionally, an aqueous solvent.

Further preferably, the substrate for synthesizing RNA comprises: one of a nucleoside monophosphate and a nucleoside triphosphate, or combinations thereof.

Further preferably, the substrate for synthesizing a protein comprises: twenty natural amino acids and non-natural amino acid.

Further preferably, the magnesium ions are derived from a magnesium ion source, and the magnesium ion source is selected from the group consisting of: magnesium acetate, magnesium glutamate, or combinations thereof.

Further preferably, the potassium ions are derived from a potassium ion source, and the potassium ion source is selected from the group consisting of: potassium acetate, potassium glutamate, or combinations thereof.

Further preferably, the energy regeneration system is selected from the group consisting of: a creatine phosphate/creatine kinase system, a glycolysis pathway and intermediate product energy system thereof, or combinations thereof.

Further preferably, the energy regeneration system comprises a glucose/phosphate system, and the phosphate is selected from the group consisting of: tripotassium phosphate, triammonium phosphate, trisodium phosphate, dipotassium hydrogen phosphate, diammonium hydrogen phosphate, disodium hydrogen phosphate, potassium dihydrogen phosphate, ammonium dihydrogen phosphate, sodium dihydrogen phosphate, or combinations thereof.

Further preferably, the buffer is selected from the group consisting of: 4-(2-hydroxyethyl)-1-piperazineethanesulfonic acid, tris(hydroxymethyl)aminomethane, or combinations thereof.

Further preferably, the *in vitro* protein synthesis system comprises polyethylene glycol (PEG) or an analog thereof. The concentration of polyethylene glycol or an analog thereof is not particularly limited. Typically, the concentration (w/v) of polyethylene glycol or an analog thereof is 0.1-8%, preferably 0.5-4%, more preferably 1-2%, based on the total weight of the protein synthesis system. Representative PEG is selected from the group consisting of: PEG3000, PEG3350, PEG6000, PEG8000, or combinations thereof.

Further preferably, the polyethylene glycol comprises polyethylene glycol with a molecular weight (Da) of 200-10000, such as PEG200, 400, 1500, 2000, 4000, 6000, 8000, 10000, and the like, preferably polyethylene glycol with a molecular weight of 3000-10000.

In the present invention, the RNA polymerase is not particularly limited and may be selected from one or more RNA polymerases. Typically, the RNA polymerase is T7 RNA polymerase.

An optional embodiment is that the *in vitro* protein synthesis system provided by the present invention comprises: a cell extract, 4-(2-hydroxyethyl)-1-piperazineethanesulfonic acid, potassium acetate, magnesium acetate, adenosine triphosphate (ATP), guanosine triphosphate (GTP), cytidine triphosphate (CTP), thymidine triphosphate (TTP), an amino acid mixture, creatine phosphate, dithiothreitol (DTT), creatine kinase, and an RNA polymerase.

In the present invention, the cell extract does not contain intact cells. Typically, the cell extract comprises ribosomes for protein translation, aminoacyl-tRNA synthetases, initiation factors and elongation factors required for protein synthesis, and termination release factors. In addition, the cell extract also contains some other proteins derived from the cytoplasm of the cell, especially soluble proteins.

In the present invention, the proportion of the cell extract in the *in vitro* cell-free protein synthesis system is not particularly limited. Typically, the cell extract accounts for 20-70% of the *in vitro* cell-free protein synthesis system, preferably 30-60%, more preferably 40-50%.

In the present invention, the protein content of the cell extract is 20-100 mg/mL, preferably 50-100 mg/mL. The method for determining the protein content is the Coomassie brilliant blue assay method.

The present invention also provides a vector or a vector combination, wherein the vector comprises the nucleic acid construct of the present invention. Preferably, the vector is selected from: a bacterial plasmid, a bacteriophage, a yeast plasmid, an animal cell vector, a shuttle vector. The vector is a transposon vector. Methods for preparing recombinant vectors are well known to those of ordinary skill in the art. Any plasmid and vector may be used as long as it can replicate and be stably maintained in a host.

Those of ordinary skill in the art can construct expression vectors comprising the promoter and/or the gene sequence of interest of the present invention using well-known methods, including *in vitro* recombinant DNA technology, DNA synthesis technology, *in vivo* recombination technology, and the like.

### Template DNA

Template DNA refers to a nucleotide sequence encoding any target protein to be synthesized. It can be a native sequence, an artificially synthesized sequence or an artificially modified sequence. The template DNA can be used to synthesize corresponding RNA and/or proteins.

In the present invention, the preparation method of the cell extract is not particularly limited. One preferred preparation method comprises the following steps:
(i) Providing cells;
(ii) Washing the cells to obtain washed cells;
(iii) Disrupting the washed cells to obtain a crude cell extract;
(iv) Subjecting the crude cell extract to solid-liquid separation to collect the liquid fraction, which is the cell extract.

In the present invention, the solid-liquid separation method is not particularly limited. A preferred method is centrifugation.

In a preferred embodiment, the centrifugation is performed in a liquid state.

In the present invention, the centrifugation conditions are not particularly limited. A preferred centrifugal force is 5000-100000 g, more preferably 8000-30000 g.

In the present invention, the centrifugation time is not particularly limited. A preferred centrifugation time is 0.5 min to 2 h, more preferably 20 min to 50 min.

In the present invention, the centrifugation temperature is not particularly limited. Preferably, the centrifugation is carried out at 110 °C, more preferably at 2-6 °C.

In the present invention, the washing method is not particularly limited. A preferred washing method is performed with a washing solution at a pH of 7-8, more preferably 7.4. The washing solution is not particularly limited, and is typically selected from the group consisting of potassium 4-(2-hydroxyethyl)-1-piperazineethanesulfonate, potassium acetate, magnesium acetate, or combinations thereof.

In the present invention, the method of disruption is not particularly limited. Preferred methods of cell disruption include high-pressure homogenization and freeze-thaw lysis (e.g., ultra-low temperature treatment with liquid nitrogen).

The nucleoside triphosphate mixture in the *in vitro* cell-free protein synthesis system consists of adenosine triphosphate, guanosine triphosphate, cytidine triphosphate and uridine triphosphate. In the present invention, the concentration of each mononucleotide is not particularly limited, and is generally 0.5-5 mM, more preferably 1.0-2.0 mM.

The amino acid mixture in the *in vitro* cell-free protein synthesis system may comprise natural or non-natural amino acids, and may comprise D-form or L-form amino acids. Representative amino acids include (but are not limited to) the twenty natural amino acids: glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine. The concentration of each amino acid is typically 0.01-0.5 mM, preferably 0.02-0.2 mM, such as 0.05, 0.06, 0.07, 0.08 mM.

In a preferred embodiment, the *in vitro* cell-free protein synthesis system further comprises polyethylene glycol or analogs thereof. The concentration of polyethylene glycol or analogs thereof is not particularly limited. Typically, the concentration (w/v) of polyethylene glycol or an analog thereof is 0.1-8%, preferably 0.5-4%, more preferably 1-2%, based on the total weight of the biosynthesis system. Representative examples of PEG include (but are not limited to): PEG3000, PEG8000, PEG6000, and PEG3350. It should be understood that the system of the present invention may also comprise polyethylene glycol of various other molecular weights (such as PEG200, 400, 1500, 2000, 4000, 6000, 8000, 10000, and the like).

In a preferred embodiment, the *in vitro* cell-free protein synthesis system further contains sucrose. The concentration of sucrose is not particularly limited. Typically, the concentration of sucrose is 0.03-40 wt%, preferably 0.08-10 wt%, more preferably 0.1-5 wt%, based on the total weight of the protein synthesis system.

A particularly preferred *in vitro* cell-free protein synthesis system, in addition to the yeast cell extract, further comprises the following components: 22 mM 4-(2-hydroxyethyl)-1-piperazineethanesulfonic acid at pH 7.4, 30-150 mM potassium acetate, 1.0-5.0 mM magnesium acetate, 1.5-4 mM nucleoside triphosphate mixture, 0.08-0.24 mM amino acid mixture, 25 mM creatine phosphate, 1.7 mM dithiothreitol, 0.27 mg/mL creatine kinase, 1%-4% polyethylene glycol, 0.5%-2% sucrose, and 0.027-0.054 mg/mL T7 RNA polymerase.

The present invention is further elaborated below in conjunction with specific embodiments. It should be understood that these embodiments are provided solely to illustrate the present invention.

These embodiments are not intended to limit the scope of the present invention. Experimental procedures without specified conditions in the following examples are generally carried out under conventional conditions, such as those described in Molecular Cloning: A Laboratory Manual (New York: Cold Spring Harbor Laboratory Press, 1989) by Sambrook et al., or conditions recommended by the reagent manufacturers. Unless otherwise specified, all percentages and parts refer to weight percentages and weight parts. The present invention uses *Kluyveromyces lactis* (abbreviated as K. lactis or kl) in the examples, however, the same design, analysis and experimental methods are also applicable to other eukaryotic cells such as other yeasts and animal cells, as well as prokaryotic cells.

The present invention uses *Kluyveromyces lactis* (abbreviated as K. lactis) in the examples, however, the same design, analysis and experimental methods are also applicable to other eukaryotic cells such as other yeasts and animal cells, as well as prokaryotic cells. The genetic modification method in the present invention is CRISPR-Cas9 technology, but it is not limited thereto. Any known existing gene editing methods can be applied.

The *in vitro* protein synthesis reaction mixture system, also described as the *in vitro* protein synthesis reaction mixture, reaction mixture system, or reaction mixture, refers to a mixed system comprising the *in vitro* protein synthesis system and a nucleic acid template encoding a protein of interest. It can be homogeneous or heterogeneous, and may exist in liquid forms such as solution, emulsion and suspension.

The final concentrations of the components in the Protein Factory of the present invention are respectively: 80% (v/v) *Kluyveromyces lactis* extract, 15 mM glucose, 320 mM maltodextrin (molar concentration calculated as glucose monomer), 24 mM tripotassium phosphate, 1.8 mM nucleoside triphosphate mixture (a mixture of adenosine triphosphate, guanosine triphosphate, cytidine triphosphate, and uridine triphosphate, with the final concentration of each nucleoside triphosphate being 1.8 mM), 0.7 mM amino acid mixture (glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine, and histidine, with the final concentration of each amino acid being 0.7 mM), magnesium L-aspartate, 80 mM potassium acetate, 2% (w/v) polyethylene glycol 8000, 9.78 mM pH 8.0 Tris·HCl buffer, and 6% (w/v) trehalose. The preparation process of the *Kluyveromyces lactis* cell extract adopts conventional technical means, prepared by reference to the method described in CN109593656A. Briefly, the preparation steps comprise: providing an appropriate amount of raw material of *Kluyveromyces lactis* cells obtained through fermentation culture, rapidly freezing the cells with liquid nitrogen, disrupting the cells, and collecting the supernatant by centrifugation to obtain the cell extract. The protein concentration in the obtained *Kluyveromyces lactis* cell extract is 20-40 mg/mL. In the following examples, 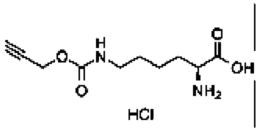 (prock, N-ε-propargyloxycarbonyl-L-lysine hydrochloride) is selected as a representative non-natural amino acid (abbreviated as ncAA), but the ncAA defined herein is not limited to prock.

### Example 1: Three Construction Methods for MaPylRS Protein

### Method 1, Long N-terminal MaPylRS:

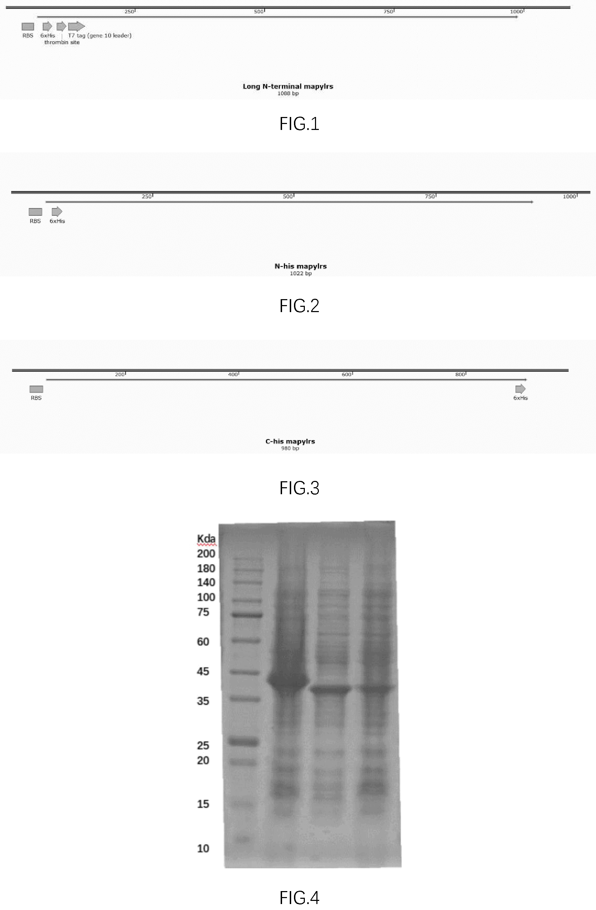

The N-terminus of the MaPylRS protein comprises a leader peptide consisting of 31 amino acids, which contains a 6×His affinity purification tag, a T7 tag, and a thrombin cleavage site (Figure 1):

### The amino acid sequence of Long N-terminal mapylrs:

MGSSHHHHHHSSGLVPRGSHMASMTGGQQMGRGSMTVKYTDAQIQRLRE
YGNGTYEQKVFEDLASRDAAFSKEMSVASTDNEKKIKGMIANPSRHGLTQLMN
DIADALVAEGFIEVRTPIFISKDALARMTITEDKPLFKQVFWIDEKRALRPMLAPN
LYSVMRDLRDHTDGPVKIFEMGSCFRKESHSGMHLEEFTMLNLVDMGPRGDAT
EVLKNYISVVMKAAGLPDYDLVQEESDVYKETIDVEINGQEVCSAAVGPHYLDA
AHDVHEPWSGAGFGLERLLTIREKYSTVKKGGASISYLNGAKIN*(SEQ ID NO:1)

Theoretical molecular weight: 34349.98 Da.

### Method 2, N-His MaPylRS:

The N-terminus of the MaPylRS protein contains only a 6×His affinity purification tag and a flexible linker (Figure 2):

MGSSHHHHHHSSGTVKYTDAQIQRLREYGNGTYEQKVFEDLASRDAAFSK
EMSVASTDNEKKIKGMIANPSRHGLTQLMNDIADALVAEGFIEVRTPIFISKDALA
RMTITEDKPLFKQVFWIDEKRALRPMLAPNLYSVMRDLRDHTDGPVKIFEMGSC

FRKESHSGMHLEEFTMLNLVDMGPRGDATEVLKNYISVVMKAAGLPDYDLVQE
ESDVYKETIDVEINGQEVCSAAVGPHYLDAAHDVHEPWSGAGFGLERLLTIREK
YSTVKKGGASISYLNGAKIN* (SEQ ID NO:2)

Theoretical molecular weight of N-His MaPylRS: 32091.35 Da.

### Method 3, C-His MaPylRS:

The N-terminus of the MaPylRS protein has one more Gly than the reported sequence, and the C-terminus has a 6×His affinity purification tag (Figure 3):

MGTVKYTDAQIQRLREYGNGTYEQKVFEDLASRDAAFSKEMSVASTDNEK
KIKGMIANPSRHGLTQLMNDIADALVAEGFIEVRTPIFISKDALARMTITEDKPLF
KQVFWIDEKRALRPMLAPNLYSVMRDLRDHTDGPVKIFEMGSCFRKESHSGMH
LEEFTMLNLVDMGPRGDATEVLKNYISVVMKAAGLPDYDLVQEESDVYKETID
VEINGQEVCSAAVGPHYLDAAHDVHEPWSGAGFGLERLLTIREKYSTVKKGGAS
ISYLNGAKINHHHHHH* (SEQ ID NO:3)

Theoretical molecular weight of C-His MaPylRS: 31685.98 Da.

Method 4, compared with Long N-terminal MaPylRS, the His tag is removed:

MGSSSGLVPRGSHMASMTGGQQMGRGSMTVKYTDAQIQRLREYGNGTY
EQKVFEDLASRDAAFSKEMSVASTDNEKKIKGMIANPSRHGLTQLMNDIADALV
AEGFIEVRTPIFISKDALARMTITEDKPLFKQVFWIDEKRALRPMLAPNLYSVMRD
LRDHTDGPVKIFEMGSCFRKESHSGMHLEEFTMLNLVDMGPRGDATEVLKNYIS
VVMKAAGLPDYDLVQEESDVYKETIDVEINGQEVCSAAVGPHYLDAAHDVHEP
WSGAGFGLERLLTIREKYSTVKKGGASISYLNGAKIN (SEQ ID NO:63).

### Example 2: Construction of Expression Plasmids

### Long N-terminal MaPylRS expression plasmid:

The full-length MaPylRS gene (WP_015505008) was synthesized by Sangon Biotech, wherein the codons were optimized for expression in *Escherichia coli.* The synthesized gene sequence is as follows:

### Nde I

CATATGGCTAGCATGACTGGTGGACAGCAAATGGGTCGCGGATCCATGACCG
TTAAATACACCGATGCGCAGATCCAGCGTCTGCGTGAATACGGTAACGGTACCTA
CGAACAGAAAGTTTTCGAAGATCTGGCGAGCCGTGATGCGGCGTTCTCTAAAGA

AATGAGCGTTGCTTCTACCGATAACGAAAAGAAAATCAAAGGTATGATCGCGAA
CCCGAGCCGTCACGGCCTGACCCAGCTGATGAACGATATCGCGGATGCTCTGGTT
GCTGAAGGTTTCATCGAAGTTCGTACCCCGATCTTCATCTCTAAAGATGCGCTGG
CGCGTATGACCATCACCGAAGATAAACCGCTGTTCAAACAGGTTTTCTGGATCGA
TGAAAAACGTGCGCTGCGTCCGATGCTGGCGCCGAACCTGTATAGCGTTATGCGT
GATCTGCGTGATCACACCGATGGTCCGGTGAAAATCTTCGAAATGGGCAGCTGCT
TCCGTAAAGAAAGCCACTCTGGTATGCACCTGGAAGAATTCACCATGCTGAACCT
GGTTGATATGGGTCCGCGTGGTGATGCGACCGAAGTTCTGAAAAACTACATCAGC
GTGGTTATGAAAGCTGCGGGTCTGCCGGATTACGATCTGGTTCAGGAAGAAAGC
GATGTTTACAAAGAAACCATCGATGTTGAAATCAACGGCCAGGAAGTTTGCAGC
GCGGCGGTTGGTCCGCATTATCTGGATGCGGCGCACGATGTTCACGAACCGTGGT
CTGGCGCTGGCTTCGGTCTGGAACGTCTGCTGACCATCCGTGAAAAATACTCTAC
CGTTAAAAAAGGTGGTGCGAGCATCAGCTACCTGAACGGCGCGAAAATCAACTA
ACTCGAG (SEQ ID NO:4) .

### Xho I

The gene was inserted into the pET28a vector through NdeI/XhoI restriction sites.

The N-His MaPylRS and C-His MaPylRS expression vectors were modified on the basis of the pET28a-long-terminal MaPylRS by PCR method. The primers used are respectively:
N-his mapylrs
N-his F: CATCACAGCAGCGGCACCGTTAAATACACCGATGCGCAG (SEQ ID NO:5)
N-his R: GGTGTATTTAACGGTGCCGCTGCTGTGATGATGATGATG (SEQ ID NO:6)
C-his mapylrs:
   C-his vector R:
      GGTGTATTTAACGGTGCCCATGGTATATCTCCTTCTTAAAGttaaac (SEQ ID NO:7)
   C-his vector F: GCGCGAAAATCAACCACCACCACCACCACCACTGag (SEQ ID NO:8)
   C-his F: GAGATATACCATGGGCACCGTTAAATACACCGATGCGCAG (SEQ ID NO:9)
   C-his R: TGGTGGTGGTGGTGGTGGTTGATTTTCGCGCCGTTCAGG (SEQ ID NO:10)

The amplified product was digested with DnpI, then ligated and transformed into competent DH5α cells. Plasmids were subsequently extracted and subjected to sequencing.

### Example 3

1) A single bacterial colony was picked and inoculated into 100 mL LB medium containing 100 mg/L kanamycin, followed by incubation at 37°C overnight.
2) On the next day, the culture was inoculated into fresh LB medium containing 100 mg/L kanamycin at a dilution ratio of 1:100, and cultivated at 37 °C until OD600 ≈ 0.6. IPTG was then added to a final concentration of 0.1 mM , and expression was induced at 16°C for 20 hours.
3) Bacterial cells were harvested by centrifugation at 5,000 rpm for 20 minutes at 4 °C. The cell pellet was resuspended in lysis buffer (25 mM Tris-HCl, 500 mM NaCl, 25 mM imidazole, 5 mM β-mercaptoethanol, 1 mM PMSF, 0.1% Triton X-100) at a ratio of 100 mL lysis buffer per 20 grams of wet bacterial cells. Cells were lysed using a high-pressure homogenizer.
4) The lysate was centrifuged twice at 20,000 rpm for 20 minutes at 4 °C to thoroughly remove bacterial debris. The resulting supernatant was subjected to purification of the target protein using a HisTrap affinity chromatography column. Buffer A: 25 mM TrisHCl (pH 7.8), 500 mM NaCl, 25 mM imidazole. Buffer B: 25 mM TrisHCl (pH 7.8), 150 mM NaCl, 500 mM imidazole.
5) After the cell lysate supernatant was loaded onto the affinity chromatography column, the column was rinsed with 10 column volumes (cv) of Buffer A. Subsequently, the target protein was eluted with a linear gradient from 0% to 100% of Buffer B over 10 column volumes.
6) Fractions containing the target protein were collected and pooled, and the sample was concentrated using an ultrafiltration centrifugal tube.
7) The concentrated sample was fully dialyzed against a dialysis buffer consisting of 50% glycerol and 25 mM Hepes (pH 7.5). Afterwards, the protein concentration was measured, and the sample was aliquoted. The prepared sample can be stored at -80 °C for at least one year. It can be seen from Figure 4 that the lysate supernatants after induction expression of the first three forms of recombinant mapylrs are obtained. The mapylrs protein with a longer N-terminal exhibited higher expression level and better solubility. The protein concentration can reach up to 120 mg/mL (3.4 mM).

### Example 4 Comparison of Activity of Purified Proteins Obtained by Three Construction Methods

The concentrations of the three proteins were determined using a Nanodrop, and the concentrations of the three proteins were adjusted to the same using DNase-free and RNase-free ddH₂O.

Establishment of the expression system containing non-natural amino acids:
100 µL of Protein Factory,
1 µL of 500 mM Prock (non-natural amino acid),
10 µL of unpurified *in vitro* transcription product of matRNA_{CUA}^{pyl},
3 µL of PCR product of GFP-TAG-RFP dual fluorescent reporter gene,
Purified Mapylrs proteins of different forms with a final concentration of 5 µM.

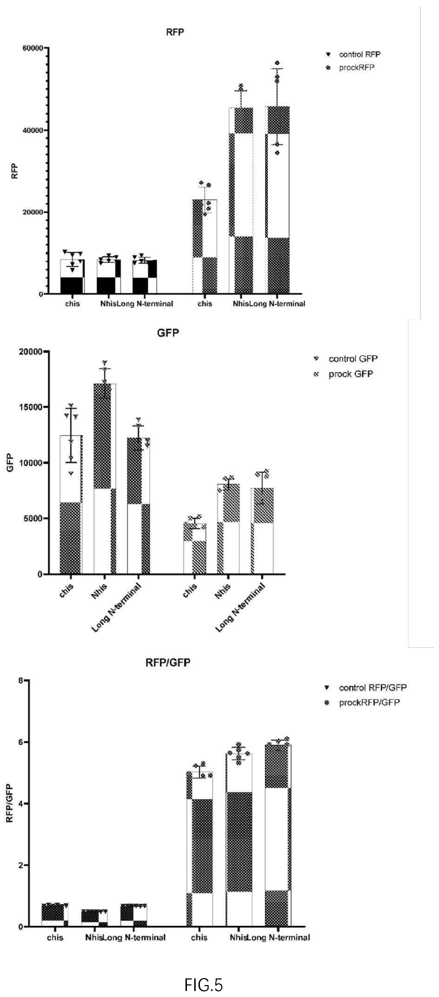

The fluorescence intensity of GFP was detected at an excitation wavelength of 485 nm and an emission wavelength of 535 nm (Ex485 nm/Em535 nm). The fluorescence intensity of RFP was detected at an excitation wavelength of 535 nm and an emission wavelength of 595 nm (Ex535 nm/Em595 nm). The incorporation efficiency of non-natural amino acids was determined according to the fluorescence intensity of RFP and the RFP/GFP ratio (see Figure 5 for details).

As can be seen from Figure 5, based on RFP/GFP, all three forms of Mapylrs are capable of achieving efficient incorporation of the non-natural amino acid and exhibit high activities. Among them, the Long-N-terminal mapylrs with an N-terminal tag shows superior activity because the N-terminal tag stabilizes the protein structure.

### Example 5: Detection of the Effect of the Purified Native Mapylrs (i.e., Non-Recombinant Mapylrs) and the N-Terminal Tagged Mapylrs on the Activity of Non-Natural Amino Acid Incorporation

The activities of native Mapylrs, Long-N-terminal mapylrs and N-his maplys were compared. The activity assay was performed by adopting the dual fluorescent protein expression method as described in Example 4.

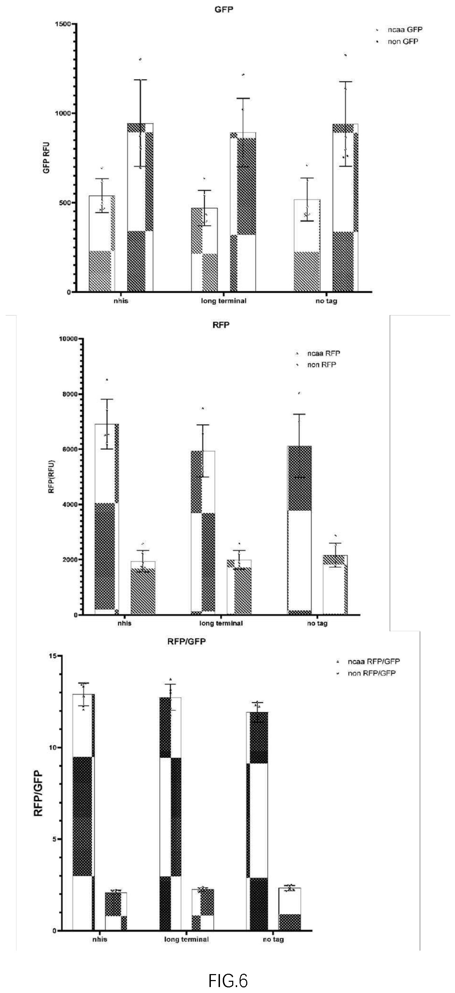

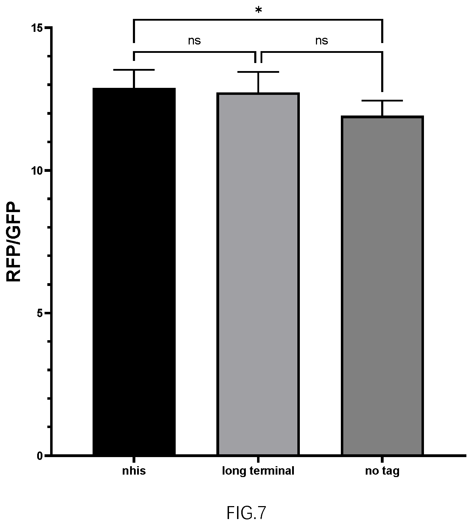

As shown in Figure 6 and Figure 7, compared with native Mapylrs (labeled as no tag in the figures), both Long-N-terminal mapylrs and N-his maplys exhibit improved activity in incorporating non-natural amino acids, indicating that the recombinant Mapylrs modified by the present invention exhibit significant improvement in the incorporation of non-natural amino acids relative to native Mapylrs.

To overcome the drawbacks of existing non-natural amino acid insertion systems, which require manual exogenous addition of MaPylRS protein or transformation/transfection to introduce plasmids carrying its expression construct, the present invention further discloses integrating MaPylRS protein into the cellular genome by gene editing technology. A strain capable of stably expressing MaPylRS protein at an appropriate level is constructed, thereby establishing a simple and efficient non-natural amino acid insertion system without the need for exogenous MaPylRS supplementation.

Hereinafter, only the Long-N-terminal mapylrs with the his-tag removed (i.e., using the fourth structure) is taken as an example to verify the integration of the MaPylRS protein into the cell genome, which shall not limit other MaPylRS of the present invention.

### Example 6: Insertion of the MaPylRS Expression Cassette (i.e., the Expression Cassette of the Fourth Structure, the Same Below) Near KlLysl-5 by CRISPR-Cas9 Technology

### (1) Retrieval of KlLys1-5 Sequence and Determination of CRISPR gRNA Sequence

In order not to affect the normal expression of other genes of *Kluyveromyces lactis,* the MaPylRS expression construct was inserted into the vicinity of tDNA *KlLysl-5* of *Kluyveromyces lactis.* After expression, the expressed product binds to non-natural amino acids and performs site-specific insertion.
i. The *Lys1-5* sequence of *K. lactis,* tRNA-Lys-CTT-1-5, was retrieved from t he website: http://gtrnadb.ucsc.edu/GtRNAdb2/genomes/eukaryota/Kluy_lact_NRRL_Y _1140/Kluy_lact_NRRL_Y_1140-gene-list.html. This gene was designated as *KlLys1* -5 in the present invention, which is located at positions 856274 to 856346 on Ch romosome E.
ii. PAM sequences (NGG) were searched within the range of 500 bp upstream and downstream of *KlLys1-5.* Finally, the PAM sequence located downstream of the gene (positions 856876 to 856878 on Chromosome E) was selected, and the *KlLysl-5* gRNA sequence was determined as GTTCCCATTGATCCCATATC (SEQ ID NO: 11), which corresponds to positions 856856 to 856875 on Chromosome E.

### (2) Construction of KlLys1-5 CRISPR-Cas9 Plasmid

Two 24 nt primers required for vector construction were designed according to the obtained gRNA sequence. gRNA-F1: AATCGTTCCCATTGATCCCATATC (SEQ ID NO: 12); gRNA-R1: AAACGATATGGGATCAATGGGAAC (SEQ ID NO: 13). The synthesized primers were diluted to 10 µM. 10 µL of gRNA-F1 and 10 µL of gRNA-R1 were added into a PCR tube respectively. The mixture was centrifuged to the bottom of the tube, and then subjected to annealing following the procedures below:
95 °C for 3 min; 72 °C for 30 s; 65 °C for 2 min; 60 °C for 2 min; 55 °C for 2 min; 50 °C for 2 min; 16 °C for 2 min. A ligation reaction was carried out subsequently.
A. Reaction system: 1 µL of 10× Buffer, 20-50 ng of plasmid, 1 µL of annealed product, 0.2 µL of enzyme, and sterile water was supplemented to a total volume of 10 µL.
B. Reaction procedure: 16°C for 60 min.

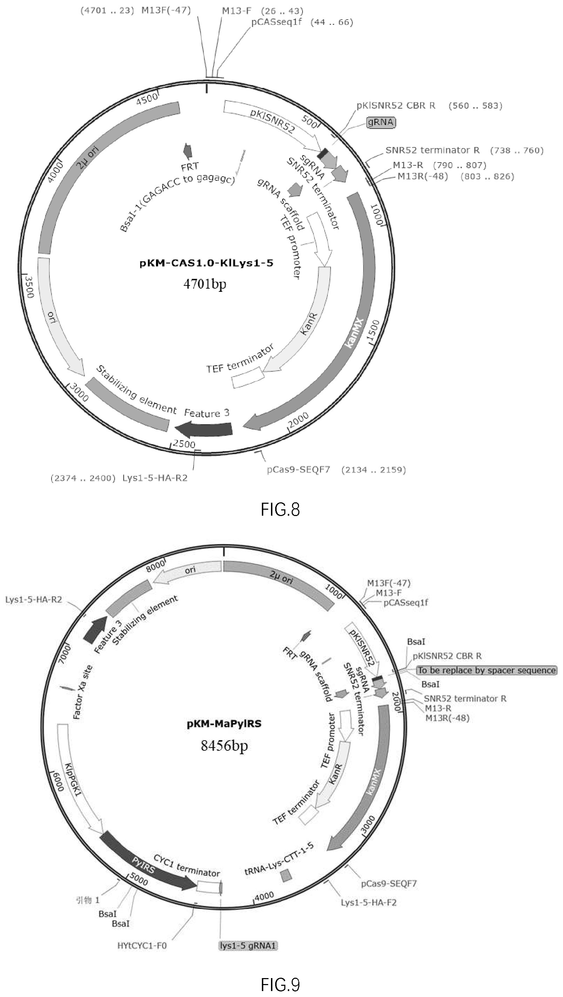

50 µL of commercially available competent cells of *E. coli* DH5α were mixed with all ligation products. The transformation procedure was performed in accordance with the product manual. Screening was conducted on LB plates containing 50 mg/L kanamycin, followed by overnight incubation. Five single colonies were picked and cultured with shaking in liquid LB medium. After positive clones were confirmed by sequencing, the plasmid was extracted and preserved, and named pKM-CAS1.0-Kllys1-5 (see Figure 8).

### (3) Construction and Amplification of Donor DNA

A donor was first constructed, which contained the homologous recombination sequences targeting the *KlLys1-5* locus and the MaPylRS expression construct. The promoter of MaPylRS is the KlPGK1 promoter, and the terminator is the ScCYC1 terminator. The Donor DNA construction and transformation method is as follows:
iii. A plasmid carrying the MaPylRS expression cassette was synthesized and designated as pKM-MaPylRS (see Figure 9). Using this plasmid as the template, PCR amplification was performed with primer PF1: AATGTTCCCATTGATCCCATATCCTTCGAGCGTCCCAAAACC (SEQ ID NO: 14) and primer PR1: TTCAGTTCAAAAACGCCCCGTTCCTCATCACTAGAAG (SEQ ID NO: 15) to obtain the MaPylRS expression cassette fragment.
iv. Using the episomal plasmid of *Kluyveromyces lactis* as the template, PCR amplification was carried out with primer PF2: GTTATTAATGTCGTGTGCCATAGGT (SEQ ID NO: 16) and primer PR2: AGGTTTTGGGACGCTCGAAGGATATGGGATCAATGGGAA (SEQ ID NO: 17) to obtain the homologous arm 1 fragment of the *KlLys1-5* locus. Using the episomal plasmid of *Kluyveromyces lactis* as the template, PCR amplification was performed with primer PF3: TTCTAGTGATGAGGAACGGGGCGTTTTTGAACTGAATTTCG (SEQ ID NO: 18) and primer PR3: CAGCATAGCATTTGAGTATTGTG (SEQ ID NO: 19) to obtain the homologous arm 2 fragment of the *KlLysl-5* locus.
v. The above three PCR product fragments were mixed and diluted 100-fold to serve as the template. A second round of PCR amplification was performed using primer PF4: ATAGGTCAATTAATAATATGCCAGCAAT (SEQ ID NO: 20) and primer PR4: GGGGAGCATAGCATTCAAAAACTTC (SEQ ID NO: 21). The three fragments were ligated together via overlap extension PCR to generate linear donor DNA. After verification by sequencing, the prepared donor DNA was stored in a refrigerator at -20°C.

### (4) Preparation of High-Efficiency Competent Cells of Kluyveromyces lactis and Transformation

### Preparation of Yeast Competent Cells

The strain of *Kluyveromyces lactis* was streaked on solid YPD medium, and a single colony was picked and cultured with shaking in 25 mL of 2× YPD liquid medium overnight. 2 mL of the resultant culture was transferred into 50 mL of 2× YPD liquid medium and cultured with shaking for another 2 to 8 hours. Yeast cells were collected by centrifugation at 3000 g for 5 min at 20°C, resuspended in 500 µL of sterile water, and centrifuged again under the same conditions to harvest cells. A competent cell solution containing 5% (v/v) glycerol and 10% (v/v) DMSO was prepared, and the collected yeast cells were fully resuspended in 500 µL of the solution. The cell suspension was aliquoted into 1.5 mL centrifuge tubes at 50 µL per tube and stored at -80 °C.

### Yeast DNA Transformation

The competent cells were thawed on ice for 30 seconds. 200 ng of pKM-CAS1.0-KlLys1-5 plasmid and 2000 ng of donor DNA were added sequentially. Electroporation was performed at 1.5 kV for 5 mS. Immediately after electroporation, 1 mL of YPD liquid medium was added, and the mixture was incubated for 2 to 3 hours. 200 µL of the culture was spread onto solid YPD medium supplemented with 200 µg/mL G418 and incubated for 2 to 3 days until single colonies formed.

### (5) Identification of Positive Gene-Edited Clones

Fifty to sixty single colonies were picked from the transformed plates of *Kluyveromyces lactis.* Each single colony was resuspended in 5 µL of yeast lysate (Takara Mighty Prep Reagent for DNA). Use the cell lysate as the template, and perform PCR amplification with the following primer pairs respectively: Ma R1 (internal primer for MaPylRS sequence): ATCTCTTACTTGAACGGTGCTA (SEQ ID NO: 22); 1-5 F1 (5' external primer for *KlLysl-5* donor DNA): GGTTATCCATTCAGGCAATGAAG (SEQ ID NO: 23), and Ma F1 (internal primer for MaPylRS sequence): CCATGTGAGAACCTCTTGG (SEQ ID NO: 24); 1-5 R1 (5' external primer for *KlLysl-5* donor DNA): CAGCATAGCATTTGAGTATTGTG (SEQ ID NO: 25) to detect the CRISPR insertion at the *KlLysl-5* locus. The presence of positive bands indicates successful insertion of the MaPylRS sequence into the target site.

### Example 7: Insertion of the MaPylRS Expression Cassette Near KlglpA by CRISPR-Cas9 Technology

### (1) Retrieval of KlglpA Sequence and Determination of CRISPR gRNA Sequence

In order not to affect the normal expression of other genes of *Kluyveromyces lactis,* the MaPylRS expression construct was inserted into the vicinity of tDNA *KlglpA* of *Kluyveromyces lactis.* After expression, the expressed product binds to non-natural amino acids and performs site-specific insertion.
i. The *glpA* sequence of *K. lactis,* glycerol-3-phosphate dehydrogenase, was retrieved from the website: https://www.genome-jp/kegg/kegg2.html. This gene was designated as *KlglpA* in the present invention, which is located at positions 33084 to 35012 on Chromosome A.
ii. PAM sequences (NGG) were searched within the region ranging from 1000 bp to 2000 bp upstream of *KlglpA.* Finally, the PAM sequence located downstream of the gene (positions 31956 to 31958 on Chromosome A) was selected, and the *KlglpA* gRNA sequence was determined as GAAGTAACTCTAGCCATCGG (SEQ ID NO: 26), which corresponds to positions 31936 to 31955 on Chromosome A.

### (2) Construction of KlglpA CRISPR-Cas9 Plasmid

Two 24 nt primers required for vector construction were designed according to the obtained gRNA sequence. gRNA-F2: AATCGAAGTAACTCTAGCCATCGG (SEQ ID NO: 27); gRNA-R2: AAACCCGATGGCTAGAGTTACTTC (SEQ ID NO: 28). The synthesized primers were diluted to 10 µM. 10 µL of gRNA-F2 and 10 µL of gRNA-R2 were added into a PCR tube respectively. The mixture was centrifuged to the bottom of the tube, and then subjected to annealing following the procedures below:
95 °C for 3 min; 72 °C for 30 s; 65 °C for 2 min; 60 °C for 2 min; 55 °C for 2 min; 50 °C for 2 min; 16 °C for 2 min.

A ligation reaction was carried out subsequently.
A. Reaction system: 1 µL of 10× Buffer, 20-50 ng of plasmid, 1 µL of annealed product, 0.2 µL of enzyme, and sterile water was supplemented to a total volume of 10 µL.
B. Reaction procedure: 16 °C for 60 min.

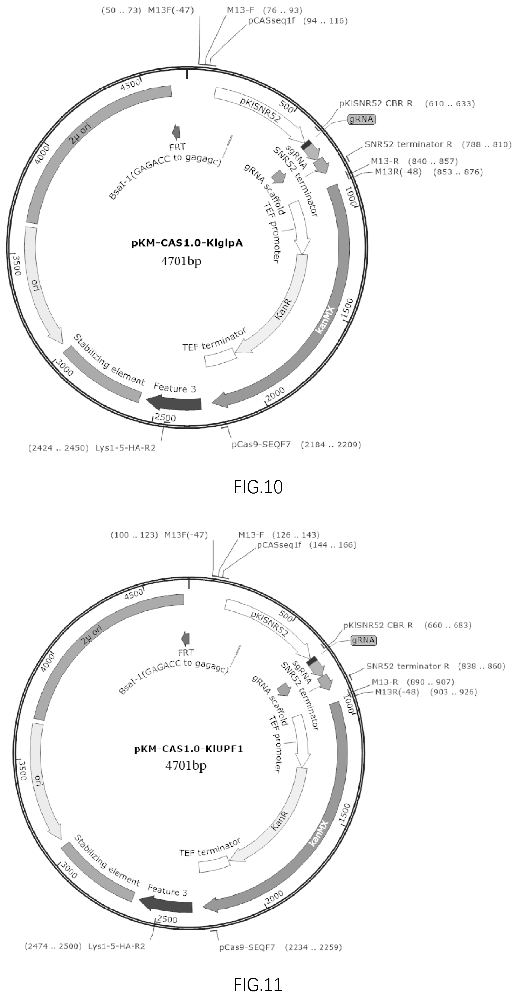

50 µL of commercially available competent cells of *E. coli* DH5α were mixed with all ligation products. The transformation procedure was performed in accordance with the product manual. Screening was conducted on LB plates containing 50 mg/L kanamycin, followed by overnight incubation. Five single colonies were picked and cultured with shaking in liquid LB medium. After positive clones were confirmed by sequencing, the plasmid was extracted and preserved, and named pKM-CAS1.0-KlglpA (see Figure 10).

### (3) Construction and Amplification of Donor DNA

A donor was first constructed, which contained the homologous recombination sequences targeting the *KlglpA* locus and the MaPylRS expression construct. The promoter of MaPylRS is the KlPGK1 promoter, and the terminator is the ScCYC1 terminator. The Donor DNA construction and transformation method is as follows:
iii. A plasmid carrying the MaPylRS expression cassette was synthesized. Using this plasmid as the template, PCR amplification was performed with primer PF5: CCATCAGTTACGGTAGATTCTCCAGTGCCTACGTTCCTCATCACTAGAAG (SEQ ID NO: 29) and primer PR5: TGTTTTGCGCTTGGTTTTCTTTGTGGAGAAATTTCTTCGAGCGTCCCAAA (SEQ ID NO: 30) to obtain the MaPylRS expression cassette fragment.
iv. Using the episomal plasmid of *Kluyveromyces lactis* as the template, PCR amplification was carried out with primer PF6: AAATTAAGGCAAACATACAGG (SEQ ID NO: 31) and primer PR6: CAACAGTTCGGCTTCTAGTGATGAGGAACGTAGGCACTGGAGAATCTACC (SEQ ID NO: 32) to obtain the homologous arm 1 fragment of the *KlglpA* locus. Using the episomal plasmid of *Kluyveromyces lactis* as the template, PCR amplification was performed with primer PF7: GCTTGAGAAGGTTTTGGGACGCTCGAAGAAATTTCTCCACAAAGAAAACC (SEQ ID NO: 33) and primer PR7: GACCTTTTATTTTGTCACCG (SEQ ID NO: 34) to obtain the homologous arm 2 fragment of the *KlglpA* locus.
v. The above three PCR product fragments were mixed and diluted 100-fold to serve as the template. A second round of PCR amplification was performed using primer PF8: ATATCGGATGACATGCAGCAA (SEQ ID NO: 35) and primer PR8: TTGTGTACCAAAACTTTCACGG (SEQ ID NO: 36). The three fragments were ligated together via overlap extension PCR to generate linear donor DNA. After verification by sequencing, the prepared donor DNA was stored in a refrigerator at -20°C.

### (4) Preparation of High-Efficiency Competent Cells of Kluyveromyces lactis and Transformation

### Preparation of Yeast Competent Cells

The strain of *Kluyveromyces lactis* was streaked on solid YPD medium, and a single colony was picked and cultured with shaking in 25 mL of 2× YPD liquid medium overnight. 2 mL of the resultant culture was transferred into 50 mL of 2× YPD liquid medium and cultured with shaking for another 2 to 8 hours. Yeast cells were collected by centrifugation at 3000 g for 5 min at 20°C, resuspended in 500 µL of sterile water, and centrifuged again under the same conditions to harvest cells. A competent cell solution containing 5% (v/v) glycerol and 10% (v/v) DMSO was prepared, and the collected yeast cells were fully resuspended in 500 µL of the solution. The cell suspension was aliquoted into 1.5 mL centrifuge tubes at 50 µL per tube and stored at -80 °C.

### Yeast DNA Transformation

The competent cells were thawed on ice for 30 seconds. 200 ng of pKM-CAS1.0-KlglpA plasmid and 2000 ng of donor DNA were added sequentially. Electroporation was performed at 1.5 kV for 5 mS. Immediately after electroporation, 1 mL of YPD liquid medium was added, and the mixture was incubated for 2 to 3 hours. 200 µL of the culture was spread onto solid YPD medium supplemented with 200 µg/mL G418 and incubated for 2 to 3 days until single colonies formed.

### (5) Identification of Positive Gene-Edited Clones

Fifty to sixty single colonies were picked from the transformed plates of *Kluyveromyces lactis.* Each single colony was resuspended in 5 µL of yeast lysate (Takara Mighty Prep Reagent for DNA). Use the cell lysate as the template, and perform PCR amplification with following primer pairs respectively: Ma R2 (internal primer for MaPylRS sequence): CCATGTGAGAACCTCTTGG (SEQ ID NO: 37); glpA F1 (5' external primer for KlglpA donor DNA): AAATTAAGGCAAACATACAGG (SEQ ID NO: 38), and Ma F2 (internal primer for MaPylRS sequence): ATCTCTTACTTGAACGGTGCTA (SEQ ID NO: 39); glpA R1 (3' external primer for KlglpA donor DNA): GACCTTTTATTTTGTCACCG (SEQ ID NO: 40) to detect the CRISPR insertion at the *KlglpA* locus. The presence of positive bands indicates successful insertion of the MaPylRS sequence into the target site.

### Example 8: Knockout of KlUPF1 and Replacement with the MaPylRS Expression Cassette by CRISPR-Cas9 Technology

### (1) Retrieval of KlUPF1 Sequence and Determination of CRISPR gRNA Sequence

It has been reported in literatures that yeast UPF1 protein participates in mRNA degradation, and deletion of this gene can slow down the degradation of immature mRNA transcripts. Accordingly, the *KlUPF1* gene was completely knocked out and replaced with the MaPylRS sequence in the present invention, so as to accumulate transcripts for non-natural amino acids and improve the efficiency of non-natural amino acid incorporation.
i. The *UPF1* gene sequence of *Saccharomyces cerevisiae, ScUPF1,* was retrieved from http://www.yeastgenome.org/. BLAST analysis was performed against the NCBI database using the *UPF1* gene sequence, and the homologous *UPF1* gene sequence of *Kluyveromyces lactis,* designated as *KlUPF1,* was identified. *KlUPF1* is located at positions 567908 to 570817 on Chromosome B.
ii. The PAM sequence (NGG) was searched at both ends of the *KlUPF1* gene, and the gRNA sequences were determined. The principles for gRNA selection were set as follows: moderate GC content, with the standard of the present invention being a GC content of 40%-60%; and avoidance of the presence of poly T structures. Finally, the sequence of *KlUPF1* gRNA1 determined by the present invention is TTGGCAAACGCATCGTCATA (SEQ ID NO: 41) and the sequence of gRNA2 is CTTAAGGAAGTACAATGGAG (SEQ ID NO: 42).

### (2) Construction of KlUPF1 CRISPR-Cas9 Plasmid

Two 24 nt primers required for vector construction were designed according to the obtained gRNA sequence. gRNA-F3: AATCTTGGCAAACGCATCGTCATA (SEQ ID NO: 43); gRNA-R3: AAACTATGACGATGCGTTTGCCAA (SEQ ID NO: 44); gRNA-F4: AATCCTTAAGGAAGTACAATGGAG (SEQ ID NO: 45); gRNA-R4: AAACCTCCATTGTACTTCCTTAAG (SEQ ID NO: 46). The synthesized primers were diluted to 10 µM. 10 µL of the gRNA-F and 10 µL of gRNA-R were added into a PCR tube respectively. The mixture was centrifuged to the bottom of the tube, and then subjected to annealing following the procedures below:
95 °C for 3 min; 72 °C for 30 s; 65 °C for 2 min; 60 °C for 2 min; 55 °C for 2 min; 50 °C for 2 min; 16 °C for 2 min.

A ligation reaction was performed afterwards.
A. Reaction system: 1 µL of 10× Buffer, 20-50 ng of plasmid, 1 µL of annealed product, 0.2 µL of enzyme, and sterile water was added to reach a total volume of 10 µL.
B. Reaction procedure: 16°C for 60 min.

50 µL of commercially available competent cells of *E. coli* DH5α were mixed with all ligation products. The transformation procedure was performed in accordance with the product manual. Screening was conducted on LB plates containing 50 mg/L kanamycin, followed by overnight incubation. Five single colonies were picked and cultured with shaking in liquid LB medium. After positive clones were confirmed by sequencing, the plasmid was extracted and preserved, and named pKM-CAS1.0-KlUPF1 (see Figure 11).

### (3) Construction and Amplification of Donor DNA

A donor was constructed in the present invention, in which the *UPF1* coding sequence is replaced by the coding sequence of MaPylRS. That is, the native promoter and terminator of *UPF1* were employed for the inserted MaPylRS. The construction and amplification methods of the donor DNA are described as follows:
iii. A plasmid carrying the MaPylRS expression cassette was synthesized. Using this plasmid as the template, PCR amplification was performed with primer PF9: AGTACAATTAGAATCAAGTTTCCTTATGGGTTCTTCTTCTTCTGG (SEQ ID NO: 47) and primer PR9: TAATATTATTTAATTAATGGATTGATACGCGTTCATGTTTAGTTGATCTTAGCA CCGTTC (SEQ ID NO: 48) to obtain the coding sequence of MaPylRS.
iv. Using the episomal plasmid of *Kluyveromyces lactis* as the template, PCR amplification was conducted with primer PF10: CAATGGATACAGTTTCTCGCTA (SEQ ID NO: 49) and primer PR10: ACCAGAAGAAGAAGAACCCATAAGGAAACTTGATTCTAATTGT (SEQ ID NO: 50) to obtain the homologous arm 1 fragment of the *KlUPF1* locus. Using the episomal plasmid of *Kluyveromyces lactis* as the template, PCR amplification was then performed with primer PF11: TTGAACGGTGCTAAGATCAACTAAACATGAACGCGTATCAATC (SEQ ID NO: 51) and primer PR11: CTTCGAGACTTCCAATGATCTC (SEQ ID NO: 52) to obtain the homologous arm 2 fragment of the *KlUPF1* locus.
v. The above three PCR product fragments were mixed and diluted 100-fold to serve as the template. A second round of PCR amplification was carried out using primer PF12: GATCGTCCATTAGCTTATCTACAAATGCC (SEQ ID NO: 53) and primer PR12: GTGAGAATGCCAGACGAT (SEQ ID NO: 54). The three fragments were ligated together via overlap extension PCR to generate linear donor DNA. After verification by sequencing, the prepared donor DNA was stored in a refrigerator at -20°C.

### (4) Transformation of Kluyveromyces lactis and Identification of Positive Clones

### Preparation of Yeast Competent Cells

The *Kluyveromyces lactis* strain was streaked on solid YPD medium, and a single colony was picked and cultured with shaking in 25 mL of 2× YPD liquid medium overnight. 2 mL of the resultant culture was transferred into 50 mL of 2× YPD liquid medium and cultured with shaking for another 2 to 8 hours. Yeast cells were collected by centrifugation at 3000 g for 5 min at 20°C, resuspended in 500 µL of sterile water, and centrifuged again under the same conditions to harvest cells. A competent cell solution containing 5% (v/v) glycerol and 10% (v/v) DMSO was prepared, and the collected yeast cells were fully resuspended in 500 µL of the solution. The cell suspension was aliquoted into 1.5 mL centrifuge tubes at 50 µL per tube and stored at -80°C.

### Yeast DNA Transformation

The competent cells were thawed on ice for 30 seconds. 200 ng of pKM-CAS1.0-KlUPF1 plasmid and 2000 ng of donor DNA were added sequentially. Electroporation was performed at 1.5 kV for 5 ms. Immediately after electroporation, 1 mL of YPD liquid medium was added, and the mixture was incubated for 2 to 3 hours. 200 µL of the culture was spread onto solid YPD medium supplemented with 200 µg/mL G418 and incubated for 2 to 3 days until single colonies formed.

### (5) Identification of Positive Gene-Edited Clones

Fifty to sixty single colonies were picked from the transformed plates of *Kluyveromyces lactis.* Each single colony was resuspended in 5 µL of yeast lysate (Takara Mighty Prep Reagent for DNA). Use the cell lysate as the template, and perform PCR amplification with the following primer pairs respectively: Ma R3 (internal primer for MaPylRS sequence): GTCTCTAGAAGCCAAGTCTTC (SEQ ID NO: 55); UPF1 F1 (5' external primer for KlUPF1 donor DNA): GAACTGCCACGGGCT (SEQ ID NO: 56), and Ma F3 (internal primer for MaPylRS sequence): GCTGCTCACGACGTTCA (SEQ ID NO: 57); UPF1 R1 (3' external primer for KlUPF1 donor DNA): GCACTGTAATCAGGCAACT (SEQ ID NO: 58) to detect the CRISPR insertion at the *KlUPF1* locus. The presence of positive bands indicates successful insertion of the MaPylRS sequence into the target site.

### Example 9: Activity Assay

The genetically modified *Kluyveromyces lactis* strain was prepared into an *in vitro* transcription-translation (IVTT) system, in which a plasmid carrying dual reporter genes of green fluorescent protein (GFP) and red fluorescent protein (RFP) was added to evaluate the capability of the modified strain to incorporate non-natural amino acids at specific sites of target proteins. GFP was used to detect the overall protein expression level, while RFP is used to detect whether a non-natural amino acid is successfully incorporated at a specific position, i.e., at the stop codon TAG of GFP. If a red fluorescence signal appears, it indicates that the translation-related proteins have read through the stop codon TAG, meaning that the non-natural amino acid has been successfully incorporated; whereas the absence of a red fluorescence signal indicates that the translation-related proteins have stopped at the stop codon TAG, and the non-natural amino acid incorporation has failed.

Taking the *Kluyveromyces lactis* strain obtained in Example 6 as an example, and adjusting the promoter in the MaPylRS expression cassette, different *Kluyveromyces lactis* strains were obtained and prepared into Protein Factories.

### Establishment of expression system containing non-natural amino acids

100 µL of Protein Factory,
1 µL of 500 mM Prock (non-natural amino acid),
10 µL of unpurified *in vitro* transcription product of matRNA_{CUA}^{pyl},
3 µL of PCR product of GFP-TAG-RFP dual fluorescent reporter gene.

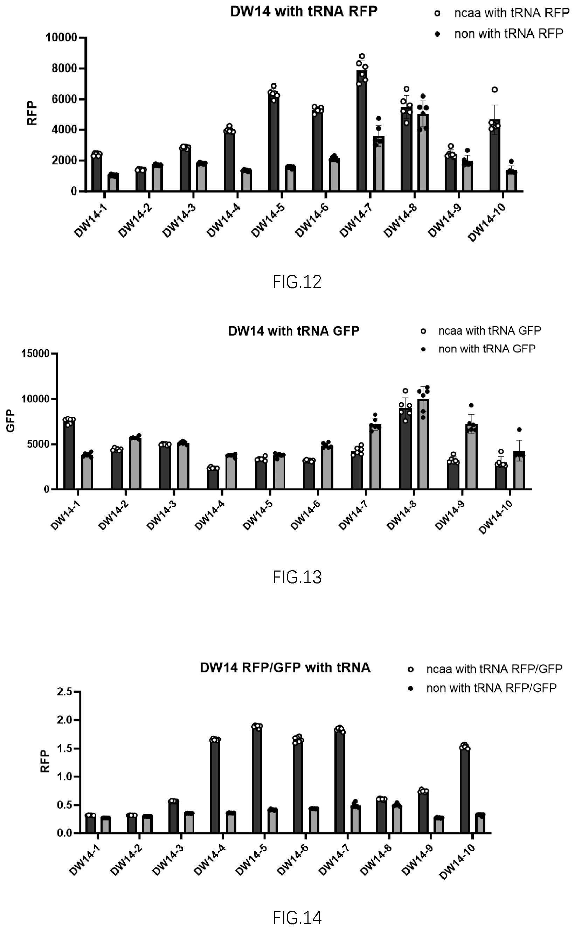

By detecting the RFP fluorescence intensity, the incorporation efficiency of non-natural amino acids was determined based on the RFP fluorescence intensity and the RFP/GFP ratio (see Figure 12 - 14 for details).

In Figures 12-14, DW14-1 and DW14-2 are two parallel experiments, with the promoter being TIF11; the promoter of DW14-3 is TEF1; DW14-4 and DW14-5 are two parallel experiments, with the promoter being ADH1; DW14-6 and DW14-7 are two parallel experiments, with the promoter being GAP1; DW14-8 and DW14-9 are two parallel experiments, with the promoter being HXK4; and the promoter of DW14-10 is PGK1.

As can be seen from Figure 12, the modified strains all achieved read-through of the stop codon TAG, demonstrating that the modified strains of the present invention are all capable of achieving non-natural amino acid incorporation. As can be seen from Figure 14, the modified strains of the present invention all exhibit high incorporation efficiency of non-natural amino acids, especially DW14-4 to DW14-7 and DW14-10, which exhibit extremely high RFP/GFP values, indicating that they all have extremely high efficiency in introducing non-natural amino acids.

### Example 10

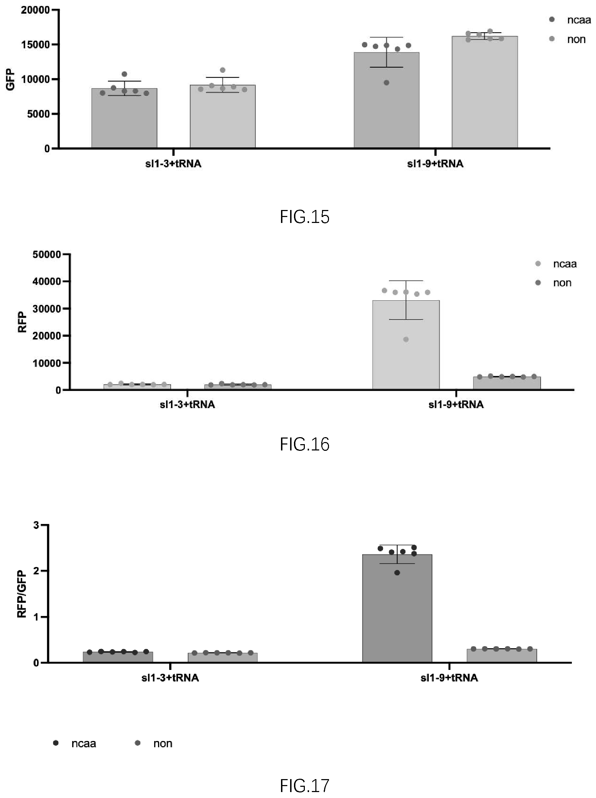

The effect on ncAA incorporation activities between the reaction system obtained after integrating the modified MaPylRS of Example 6 into yeast and the reaction system obtained after integrating the native MaPylRS (Example 5) into yeast were compared. The assay conditions were consistent with those specified in Example 9, and the results are shown in Figure 15 - 17, wherein sl-3 represents the reaction system of yeast integrated with native MaPylRS, and sl-9 represents the reaction system of yeast integrated with the modified MaPylRS of the present application. Orthogonal tRNA and Prock were supplemented in all systems, and systems without addition of non-natural amino acids (marked as non) were set as controls. It can be seen from Figure 15 - 17 that the reaction systems obtained by integrating the wild-type and modified MaPylRS into yeast cells are both capable of achieving ncAA incorporation, but the ncAA incorporation efficiency is significantly improved in the reaction system obtained after integrating the modified MaPylRS of the present invention into yeast cells.

The sequences used in the present invention are shown in Table 1 below.

**Table 1**

| structural name | SEQ ID NO | Sequence |
|---|---|---|
| The amino acid sequence of Long N-terminal mapylrs | SEQ ID NO:1 | MGSSHHHHHHSSGLVPRGSHMASMTGGQQMGRGSMTVKYTDAQIQ RLREYGNGTYEQKVFEDLASRDAAFSKEMSVASTDNEKKIKGMIANP SRHGLTQLMNDIADALVAEGFIEVRTPIFISKDALARMTITEDKPLFKQ VFWIDEKRALRPMLAPNLYSVMRDLRDHTDGPVKIFEMGSCFRKESH SGMHLEEFTMLNLVDMGPRGDATEVLKNYISVVMKAAGLPDYDLVQ EESDVYKETIDVEINGQEVCSAAVGPHYLDAAHDVHEPWSGAGFGLE RLLTIREKYSTVKKGGASISYLNGAKIN |
| The amino acid sequence of N-his mapylrs | SEQ ID NO:2 | MGSSHHHHHHSSGTVKYTDAQIQRLREYGNGTYEQKVFEDLASRDA AFSKEMSVASTDNEKKIKGMIANPSRHGLTQLMNDIADALVAEGFIEV RTPIFISKDALARMTITEDKPLFKQVFWIDEKRALRPMLAPNLYSVMR DLRDHTDGPVKIFEMGSCFRKESHSGMHLEEFTMLNLVDMGPRGDAT EVLKNYISVVMKAAGLPDYDLVQEESDVYKETIDVEINGQEVCSAAV GPHYLDAAHDVHEPWSGAGFGLERLLTIREKYSTVKKGGASISYLNG AKIN |
| The amino acid sequence of C-his mapylrs | SEQ ID NO:3 | MGTVKYTDAQIQRLREYGNGTYEQKVFEDLASRDAAFSKEMSVASTD NEKKIKGMIANPSRHGLTQLMNDIADALVAEGFIEVRTPIFISKDALAR MTITEDKPLFKQVFWIDEKRALRPMLAPNLYSVMRDLRDHTDGPVKIF EMGSCFRKESHSGMHLEEFTMLNLVDMGPRGDATEVLKNYISVVMK AAGLPDYDLVQEESDVYKETIDVEINGQEVCSAAVGPHYLDAAHDVH EPWSGAGFGLERLLTIREKYSTVKKGGASISYLNGAKINHHHHHH |
| The gene sequence of Long-N-terminal mapylrs | SEQ ID NO:4 | CATATGGCTAGCATGACTGGTGGACAGCAAATGGGTCGCGGATCCA TGACCGTTAAATACACCGATGCGCAGATCCAGCGTCTGCGTGAATA CGGTAACGGTACCTACGAACAGAAAGTTTTCGAAGATCTGGCGAGC CGTGATGCGGCGTTCTCTAAAGAAATGAGCGTTGCTTCTACCGATA ACGAAAAGAAAATCAAAGGTATGATCGCGAACCCGAGCCGTCACG GCCTGACCCAGCTGATGAACGATATCGCGGATGCTCTGGTTGCTGA AGGTTTCATCGAAGTTCGTACCCCGATCTTCATCTCTAAAGATGCG CTGGCGCGTATGACCATCACCGAAGATAAACCGCTGTTCAAACAGG TTTTCTGGATCGATGAAAAACGTGCGCTGCGTCCGATGCTGGCGCC GAACCTGTATAGCGTTATGCGTGATCTGCGTGATCACACCGATGGT CCGGTGAAAATCTTCGAAATGGGCAGCTGCTTCCGTAAAGAAAGCC ACTCTGGTATGCACCTGGAAGAATTCACCATGCTGAACCTGGTTGA TATGGGTCCGCGTGGTGATGCGACCGAAGTTCTGAAAAACTACATC AGCGTGGTTATGAAAGCTGCGGGTCTGCCGGATTACGATCTGGTTC AGGAAGAAAGCGATGTTTACAAAGAAACCATCGATGTTGAAATCA |
| | | ACGGCCAGGAAGTTTGCAGCGCGGCGGTTGGTCCGCATTATCTGGA TGCGGCGCACGATGTTCACGAACCGTGGTCTGGCGCTGGCTTCGGT CTGGAACGTCTGCTGACCATCCGTGAAAAATACTCTACCGTTAAAA AAGGTGGTGCGAGCATCAGCTACCTGAACGGCGCGAAAATCAACT AACTCGAG |
| N-his F | SEQ ID NO:5 | CATCACAGCAGCGGCACCGTTAAATACACCGATGCGCAG |
| N-his R | SEQ ID NO:6 | GGTGTATTTAACGGTGCCGCTGCTGTGATGATGATGATG |
| C-his vector R | SEQ ID NO:7 | GGTGTATTTAACGGTGCCCATGGTATATCTCCTTCTTAAAGttaaac |
| C-his vector F | SEQ ID NO:8 | GCGCGAAAATCAACCACCACCACCACCACCACTGag |
| C-his F | SEQ ID NO:9 | GAGATATACCATGGGCACCGTTAAATACACCGATGCGCAG |
| C-his R | SEQ ID NO:10 | TGGTGGTGGTGGTGGTGGTTGATTTTCGCGCCGTTCAGG |
| *KlLys1-5* gRNA sequence | SEQ ID NO:11 | GTTCCCATTGATCCCATATC |
| gRNA-F1 | SEQ ID NO:12 | AATCGTTCCCATTGATCCCATATC |
| gRNA-R1 | SEQ ID NO:13 | AAACGATATGGGATCAATGGGAAC |
| primer PF 1 | SEQ ID NO:14 | AATGTTCCCATTGATCCCATATCCTTCGAGCGTCCCAAAACC |
| primer PR1 | SEQ ID NO:15 | TTCAGTTCAAAAACGCCCCGTTCCTCATCACTAGAAG |
| primer PF2 | SEQ ID NO:16 | GTTATTAATGTCGTGTGCCATAGGT |
| primer PR2 | SEQ ID NO:17 | AGGTTTTGGGACGCTCGAAGGATATGGGATCAATGGGAA |
| primer PF3 | SEQ ID NO:18 | TTCTAGTGATGAGGAACGGGGCGTTTTTGAACTGAATTTCG |
| primer PR3 | SEQ ID NO:19 | CAGCATAGCATTTGAGTATTGTG |
| primer PF4 | SEQ ID NO:20 | ATAGGTCAATTAATAATATGCCAGCAAT |
| primer PR4 | SEQ ID NO:21 | GGGGAGCATAGCATTCAAAAACTTC |
| primer Ma R1 | SEQ ID NO:22 | ATCTCTTACTTGAACGGTGCTA |
| 1-5 F1 | SEQ ID NO:23 | GGTTATCCATTCAGGCAATGAAG |
| primer Ma F1 | SEQ ID NO:24 | CCATGTGAGAACCTCTTGG |
| 1-5 R1 | SEQ ID NO:25 | CAGCATAGCATTTGAGTATTGTG |
| *KlglpA* gRNA sequence | SEQ ID NO:26 | GAAGTAACTCTAGCCATCGG |
| gRNA-F2 | SEQ ID NO:27 | AATCGAAGTAACTCTAGCCATCGG |
| gRNA-R2 | SEQ ID NO:28 | AAACCCGATGGCTAGAGTTACTTC |
| primer PF5 | SEQ ID NO:29 | CCATCAGTTACGGTAGATTCTCCAGTGCCTACGTTCCTCATCACTAG AAG |
| primer PR5 | SEQ ID NO:30 | TGTTTTGCGCTTGGTTTTCTTTGTGGAGAAATTTCTTCGAGCGTCCC AAA |
| primer PF6 | SEQ ID NO:31 | AAATTAAGGCAAACATACAGG |
| primer PR6 | SEQ ID NO:32 | CAACAGTTCGGCTTCTAGTGATGAGGAACGTAGGCACTGGAGAATC TACC |
| primer PF7 | SEQ ID NO:33 | GCTTGAGAAGGTTTTGGGACGCTCGAAGAAATTTCTCCACAAAGAA AACC |
| primer PR7 | SEQ ID NO:34 | GACCTTTTATTTTGTCACCG |
| primer PF8 | SEQ ID NO:35 | ATATCGGATGACATGCAGCAA |
| primer PR8 | SEQ ID NO:36 | TTGTGTACCAAAACTTTCACGG |
| primer Ma R2 | SEQ ID NO:37 | CCATGTGAGAACCTCTTGG |
| glpA F1 | SEQ ID NO:38 | AAATTAAGGCAAACATACAGG |
| primer Ma F2 | SEQ ID NO:39 | ATCTCTTACTTGAACGGTGCTA |
| glpA R1 | SEQ ID NO:40 | GACCTTTTATTTTGTCACCG |
| *KlUPF1* gRNA1 sequence | SEQ ID NO:41 | TTGGCAAACGCATCGTCATA |
| *KlUPF1* gRNA2 sequence | SEQ ID NO:42 | CTTAAGGAAGTACAATGGAG |
| gRNA-F3 | SEQ ID NO:43 | AATCTTGGCAAACGCATCGTCATA |
| gRNA-R3 | SEQ ID NO:44 | AAACTATGACGATGCGTTTGCCAA |
| gRNA-F4 | SEQ ID NO:45 | AATCCTTAAGGAAGTACAATGGAG |
| gRNA-R4 | SEQ ID NO:46 | AAACCTCCATTGTACTTCCTTAAG |
| primer PF9 | SEQ ID NO:47 | AGTACAATTAGAATCAAGTTTCCTTATGGGTTCTTCTTCTTCTGG |
| primer PR9 | SEQ ID NO:48 | TAATATTATTTAATTAATGGATTGATACGCGTTCATGTTTAGTTGAT CTTAGCACCGTTC |
| primer PF10 | SEQ ID NO:49 | CAATGGATACAGTTTCTCGCTA |
| primer PR10 | SEQ ID NO:50 | ACCAGAAGAAGAAGAACCCATAAGGAAACTTGATTCTAATTGT |
| primer PF11 | SEQ ID NO:51 | TTGAACGGTGCTAAGATCAACTAAACATGAACGCGTATCAATC |
| primer PR11 | SEQ ID NO:52 | CTTCGAGACTTCCAATGATCTC |
| primer PF12 | SEQ ID NO:53 | GATCGTCCATTAGCTTATCTACAAATGCC |
| primer PR12 | SEQ ID NO:54 | GTGAGAATGCCAGACGAT |
| primer Ma R3 | SEQ ID NO:55 | GTCTCTAGAAGCCAAGTCTTC |
| UPF1 F1 | SEQ ID NO:56 | GAACTGCCACGGGCT |
| primer Ma F3 | SEQ ID NO:57 | GCTGCTCACGACGTTCA |
| UPF1 R1 | SEQ ID NO:58 | GCACTGTAATCAGGCAACT |
| T7 tag | SEQ ID NO:59 | MASMTGGQQMGR |
| Native MaPylRS | SEQ ID NO:60 | TVKYTDAQIQRLREYGNGTYEQKVFEDLASRDAAFSKEMSVASTDNE KKIKGMIANPSRHGLTQLMNDIADALVAEGFIEVRTPIFISKDALARMT ITEDKPLFKQVFWIDEKRALRPMLAPNLYSVMRDLRDHTDGPVKIFEM GSCFRKESHSGMHLEEFTMLNLVDMGPRGDATEVLKNYISVVMKAA GLPDYDLVQEESDVYKETIDVEINGQEVCSAAVGPHYLDAAHDVHEP WSGAGFGLERLLTIREKYSTVKKGGASISYLNGAKIN |
| The gene sequence of N-his mapylrs | SEQ ID NO:61 | ATGGGCAGCAGCCATCATCATCATCATCACAGCAGCGGCACCGTTA AATACACCGATGCGCAGATCCAGCGTCTGCGTGAATACGGTAACGG TACCTACGAACAGAAAGTTTTCGAAGATCTGGCGAGCCGTGATGCG GCGTTCTCTAAAGAAATGAGCGTTGCTTCTACCGATAACGAAAAGA AAATCAAAGGTATGATCGCGAACCCGAGCCGTCACGGCCTGACCC AGCTGATGAACGATATCGCGGATGCTCTGGTTGCTGAAGGTTTCAT CGAAGTTCGTACCCCGATCTTCATCTCTAAAGATGCGCTGGCGCGT ATGACCATCACCGAAGATAAACCGCTGTTCAAACAGGTTTTCTGGA TCGATGAAAAACGTGCGCTGCGTCCGATGCTGGCGCCGAACCTGTA TAGCGTTATGCGTGATCTGCGTGATCACACCGATGGTCCGGTGAAA ATCTTCGAAATGGGCAGCTGCTTCCGTAAAGAAAGCCACTCTGGTA TGCACCTGGAAGAATTCACCATGCTGAACCTGGTTGATATGGGTCC GCGTGGTGATGCGACCGAAGTTCTGAAAAACTACATCAGCGTGGTT ATGAAAGCTGCGGGTCTGCCGGATTACGATCTGGTTCAGGAAGAAA GCGATGTTTACAAAGAAACCATCGATGTTGAAATCAACGGCCAGG AAGTTTGCAGCGCGGCGGTTGGTCCGCATTATCTGGATGCGGCGCA |
| | | CGATGTTCACGAACCGTGGTCTGGCGCTGGCTTCGGTCTGGAACGT CTGCTGACCATCCGTGAAAAATACTCTACCGTTAAAAAAGGTGGTG CGAGCATCAGCTACCTGAACGGCGCGAAAATCAACTAA |
| The gene sequence of C-his mapylrs | SEQ ID NO:62 | ATGGGCACCGTTAAATACACCGATGCGCAGATCCAGCGTCTGCGTG AATACGGTAACGGTACCTACGAACAGAAAGTTTTCGAAGATCTGGC GAGCCGTGATGCGGCGTTCTCTAAAGAAATGAGCGTTGCTTCTACC GATAACGAAAAGAAAATCAAAGGTATGATCGCGAACCCGAGCCGT CACGGCCTGACCCAGCTGATGAACGATATCGCGGATGCTCTGGTTG CTGAAGGTTTCATCGAAGTTCGTACCCCGATCTTCATCTCTAAAGAT GCGCTGGCGCGTATGACCATCACCGAAGATAAACCGCTGTTCAAAC AGGTTTTCTGGATCGATGAAAAACGTGCGCTGCGTCCGATGCTGGC GCCGAACCTGTATAGCGTTATGCGTGATCTGCGTGATCACACCGAT GGTCCGGTGAAAATCTTCGAAATGGGCAGCTGCTTCCGTAAAGAAA GCCACTCTGGTATGCACCTGGAAGAATTCACCATGCTGAACCTGGT TGATATGGGTCCGCGTGGTGATGCGACCGAAGTTCTGAAAAACTAC ATCAGCGTGGTTATGAAAGCTGCGGGTCTGCCGGATTACGATCTGG TTCAGGAAGAAAGCGATGTTTACAAAGAAACCATCGATGTTGAAAT CAACGGCCAGGAAGTTTGCAGCGCGGCGGTTGGTCCGCATTATCTG GATGCGGCGCACGATGTTCACGAACCGTGGTCTGGCGCTGGCTTCG GTCTGGAACGTCTGCTGACCATCCGTGAAAAATACTCTACCGTTAA AAAAGGTGGTGCGAGCATCAGCTACCTGAACGGCGCGAAAATCAA CCACCACCACCACCACCACTGA |
| The gene sequence of Long N-terminal MaPylRS with the His tag removed | SEQ ID NO:63 | ATGGGTTCTTCTTCTTCTGGTTTGGTTCCAAGAGGTTCTCACATGGC TTCTATGACCGGTGGTCAACAAATGGGTAGAGGTTCTATGACCGTT AAGTACACCGACGCTCAAATCCAAAGATTGAGAGAATACGGTAAC GGTACCTACGAACAAAAGGTTTTCGAAGACTTGGCTTCTAGAGACG CTGCTTTCTCTAAGGAAATGTCTGTTGCTTCTACCGACAACGAAAA GAAGATCAAGGGTATGATCGCTAACCCATCTAGACACGGTTTGACC CAATTGATGAACGACATCGCTGACGCTTTGGTTGCTGAAGGTTTCA TCGAAGTTAGAACCCCAATCTTCATCTCTAAGGACGCTTTGGCTAG AATGACCATCACCGAAGACAAGCCATTGTTCAAGCAAGTTTTCTGG ATCGACGAAAAGAGAGCTTTGAGACCAATGTTGGCTCCAAACTTGT ACTCTGTTATGAGAGACTTGAGAGACCACACCGACGGTCCAGTTAA GATCTTCGAAATGGGTTCTTGTTTCAGAAAGGAATCTCACTCTGGT ATGCACTTGGAAGAATTCACCATGTTGAACTTGGTTGACATGGGTC CAAGAGGTGACGCTACCGAAGTTTTGAAGAACTACATCTCTGTTGT TATGAAGGCTGCTGGTTTGCCAGACTACGACTTGGTTCAAGAAGAA TCTGACGTTTACAAGGAAACCATCGACGTTGAAATCAACGGTCAAG AAGTTTGTTCTGCTGCTGTTGGTCCACACTACTTGGACGCTGCTCAC GACGTTCACGAACCATGGTCTGGTGCTGGTTTCGGTTTGGAAAGAT TGTTGACCATCAGAGAAAAGTACTCTACCGTTAAGAAGGGTGGTGC TTCTATCTCTTACTTGAACGGTGCTAAGATCAAC |
| The amino acid sequence of Long N-terminal MaPylRS with the His tag | SEQ ID NO:64 | MGSSSSGLVPRGSHMASMTGGQQMGRGSMTVKYTDAQIQRLREYGN GTYEQKVFEDLASRDAAFSKEMSVASTDNEKKIKGMIANPSRHGLTQ LMNDIADALVAEGFIEVRTPIFISKDALARMTITEDKPLFKQVFWIDEK RALRPMLAPNLYSVMRDLRDHTDGPVKIFEMGSCFRKESHSGMHLEE |
| removed | | FTMLNLVDMGPRGDATEVLKNYISVVMKAAGLPDYDLVQEESDVYK ETIDVEINGQEVCSAAVGPHYLDAAHDVHEPWSGAGFGLERLLTIREK YSTVKKGGASISYLNGAKIN |

Inspired by the preferred embodiments described above in accordance with the present application, and through the content of the foregoing description, those skilled in the art may make various modifications and alterations without departing from the technical concept of the present application. The technical scope of the present application shall not be limited to the contents of the specification, but shall be determined by the scope of the claims.

## Claims

1. A recombinant aminoacyl-tRNA synthetase having the structure represented by Formula I:
A1-A2-A3-A4 (I);
in Formula I, "-" is independently a bond or an amino acid linker sequence,
A1 is absent or a histidine tag;
A2 is absent or a thrombin cleavage site;
A3 is absent or a tag protein;
A4 is an aminoacyl-tRNA synthetase; and at least one of A1 ~ A3 is present; the connections of A1 ~ A4 can be either from N-terminus to C-terminus or from C-terminus to N-terminus.

2. The recombinant aminoacyl-tRNA synthetase according to claim 1, **characterized in that** the aminoacyl-tRNA synthetase is selected from a natural or mutated Pyl-tRNA synthetase (PylRS), Leu-tRNA synthetase (LeuRS), Tyr-tRNA synthetase (TyrRS), Phe-tRNA synthetase (PheRS), or Trp-tRNA synthetase (TrpRS).

3. The recombinant aminoacyl-tRNA synthetase according to claim 1 or 2, **characterized in that** the aminoacyl-tRNA synthetase is selected from a natural or mutated MaPylRS, MmPylRS, MbPylRS, EcTyrRS, MjTyrRS, EcLeuRS, ScPheRS, ScTrpRS, or BsTrpRS; preferably, the aminoacyl-tRNA synthetase comprises the sequence set forth in SEQ ID NO:60 or an active fragment thereof; further preferably, the sequence of the aminoacyl-tRNA synthetase is SEQ ID NO: 62; or is a polypeptide having ≥85%, ≥90%, ≥95%, ≥97%, ≥98%, or ≥99% or more homology to the amino acid sequence set forth in SEQ ID NO: 60, and having the same activity as the sequence of SEQ ID NO: 60.

4. The recombinant aminoacyl-tRNA synthetase according to any one of claims 1 to 3, **characterized in that** the structure of the histidine tag is n×His, wherein 1≤n≤50; preferably 2≤n≤30; further preferably, 5≤n≤20; more preferably 6≤n≤10.

5. The recombinant aminoacyl-tRNA synthetase according to any one of claims 1 to 4, **characterized in that** the his-tag is linked to the N-terminus or C-terminus of the aminoacyl-tRNA synthetase; or at the N-terminus of the aminoacyl-tRNA synthetase, the thrombin cleavage site and the tag protein are linked in the order from N-terminus to C-terminus; or at the N-terminus of the aminoacyl-tRNA synthetase, the His tag, the thrombin cleavage site, and the tag protein are linked in the order from N-terminus to C-terminus.

6. The recombinant aminoacyl-tRNA synthetase according to any one of claims 1 to 5, **characterized in that** the recombinant aminoacyl-tRNA synthetase comprises the sequence set forth in any one of SEQ ID NOs: 1 ~ 3 and SEQ ID NO: 64; or an active fragment thereof; further preferably, the amino acid sequence of the recombinant aminoacyl-tRNA synthetase is selected from any one or more of the following: SEQ ID NO: 1 ~ 3 and SEQ ID NO: 64, or is a polypeptide having ≥ 85% homology, ≥90% homology, ≥95% homology, ≥97% homology, ≥ 98% homology, or ≥99% homology to the amino acid sequence set forth in any one of SEQ ID NOs: 1 ~ 3 and SEQ ID NO: 64, and having the same activity as the sequence corresponding to the homology in SEQ ID NOs: 1 ~ 3 and SEQ ID NO: 64; or the coding sequence of the recombinant aminoacyl-tRNA synthetase is selected from any one or more of the following: SEQ ID NO: 4, SEQ ID NO: 61, SEQ ID NO: 62, SEQ ID NO: 63, or comprises the sequence set forth in any one of SEQ ID NO: 4, SEQ ID NO: 61, SEQ ID NO: 62, or SEQ ID NO: 63, or an active fragment thereof, or is a nucleotide sequence having ≥85% homology, ≥90%, ≥95% homology, ≥97% homology, ≥98% homology, or ≥99% homology to the sequence set forth in any one of SEQ ID NO: 4, SEQ ID NO: 61, SEQ ID NO: 62, or SEQ ID NO: 63, and having the same activity as the sequence corresponding to the homology in SEQ ID NO: 4, SEQ ID NO: 61, SEQ ID NO: 62, or SEQ ID NO: 63.

7. A nucleic acid construct encoding the recombinant aminoacyl-tRNA synthetase according to any one of claims 1 to 6.

8. The nucleic acid construct according to claim 7, **characterized in that** the nucleic acid construct at least comprises the structure represented by Formula II: Z1-Z2-Z3-Z4, wherein Z1 ~ Z4 are respectively elements for constituting the construct; "-" is independently a bond or a nucleotide linker sequence; Z1 is absent or a coding sequence for the histidine tag, Z2 is absent or a coding sequence for the thrombin cleavage site, Z3 is absent or a coding sequence for the tag protein, and Z4 is a coding sequence for the aminoacyl-tRNA synthetase; wherein at least one of Z1 ~ Z3 is present.

9. The nucleic acid construct according to claim 8, **characterized in that** the amino acid sequence encoded by Z1 is HHHHHH; the amino acid sequence encoded by Z2 is LVPRGS; the amino acid sequence encoded by Z3 is SEQ ID NO: 59; or Z1, Z2, and Z3 each respectively encode a sequence comprising HHHHHH, LVPRGS, and the sequence set forth in SEQ ID NO: 59, or an active fragment thereof, or are nucleotides each respectively having ≥85% homology, ≥90% homology, ≥95% homology, ≥ 97% homology, ≥ 98% homology, or ≥ 99% homology to the nucleotide sequences corresponding to those encoding HHHHHH, LVPRGS, and SEQ ID NO: 59, and each respectively having the same activity as the sequences encoding HHHHHH, LVPRGS, and SEQ ID NO: 59.

10. The nucleic acid construct according to claim 8 or 9, **characterized in that** it further comprises a promoter element,
preferably, the nucleic acid construct has the structure represented by Formula III:
Z5-Z1-Z2-Z3-Z4,
wherein Z5 is a promoter element,
more preferably, the promoter is selected from PGK1, GAP1, ADH1, HXK1, GAPDH1, TEF1, or TIF11.

11. A vector, **characterized in that** the vector comprises the nucleic acid construct according to any one of claims 7 to 10.

12. A genetically engineered strain, **characterized in that** one or more sites of the genome of the genetically engineered strain are integrated with the nucleic acid construct according to any one of claims 7 to 10; or the genetically engineered strain comprises the recombinant aminoacyl-tRNA synthetase according to any one of claims 1 to 6; or the genetically engineered strain comprises the vector according to claim 11.

13. The genetically engineered strain according to claim 12, **characterized in that** the strain is derived from mammalian cells, plant cells, yeast cells, insect cells, prokaryotic cells, or any combination thereof.

14. The genetically engineered strain according to claim 12 or 13, **characterized in that** the site is selected from Lys1-5, glpA, or UPF1.

15. The genetically engineered strain according to any one of claims 12 to 14, **characterized in that** the nucleic acid construct further comprises a terminator; preferably, the terminator is selected from CYC1, GPM1, TDH2, or ACT1.

16. A method for synthesizing a protein incorporated with a non-natural amino acid, **characterized by** using the recombinant aminoacyl-tRNA synthetase according to any one of claims 1 to 6 , or using the genetically engineered strain according to any one of claims 12 to 15 to provide the recombinant aminoacyl-tRNA synthetase.

17. A cell-free system for synthesizing a protein containing a non-natural amino acid, **characterized in that** the cell-free system at least comprises: (a) a cell extract, and (b) one or more of the recombinant aminoacyl-tRNA synthetase according to any one of claims 1 to 6, the nucleic acid construct according to any one of claims 7 to 10, or the vector according to claim 11; wherein the cell extract is derived from mammalian cells, plant cells, yeast cells, insect cells, prokaryotic cells, or any combination thereof.

18. A cell-free system for synthesizing a protein containing a non-natural amino acid, **characterized in that** the cell-free system at least comprises a cell extract, and the cell extract is derived from the genetically engineered strain according to any one of claims 12 to 15.

19. The cell-free system for synthesizing a protein containing a non-natural amino acid according to claim 17 or 18, **characterized in that** the cell-free system further comprises: a non-natural amino acid, an orthogonal tRNA, and a template comprising a gene sequence of a protein of interest, wherein a codon encoding an amino acid in the gene sequence of the protein of interest is mutated.

20. A method for preparing the genetically engineered strain according to any one of claims 12 to 15, **characterized by** introducing or integrating the nucleic acid construct according to any one of claims 7 to 10 into a cell by transformation, transfection, or gene editing technology.

21. The method for preparing the genetically engineered strain according to claim 20, **characterized in that** the nucleic acid construct according to any one of claims 7 to 10 is integrated into the genome of cells via active site integration.

22. The method for preparing the genetically engineered strain according to claim 20 or 21, **characterized in that** the nucleic acid construct further comprises a terminator; preferably, the terminator is selected from CYC1, GPM1, TDH2, or ACT1.

23. The method for preparing the genetically engineered strain according to claim 21 or 22, **characterized in that** the site is selected from Lys1-5, glpA, or UPF1.

24. A kit, **characterized in that** the kit comprises the system according to any one of claims 17 to 19.

25. A method for *in vitro* synthesis of a protein containing a non-natural amino acid, **characterized by** using the cell-free system according to any one of claims 17 to 19 or the kit according to claim 24 for preparation.
